# EUROPEAN PATENT SPECIFICATION

(11) **EP 3 272 881 B1**
(45) Date of publication and mention of the grant of the patent: **07.06.2023**
(21) Application number: 17185202.3
(22) Date of filing: 13.06.2008
(51) Int. Cl.: C12Q 1/6883, G01N 33/68, G01N 33/94

(54) **PERIPHERICAL TISSUE SAMPLE CONTAINING CELLS EXPRESSING THE 5HTR2C AND/OR ADARS AS MARKERS OF THE ALTERATION OF THE MECHANISM OF THE 5HTR2C MRNA EDITING AND ITS APPLICATIONS**
PERIPHERE GEWEBEPROBE MIT ZELLEN MIT EXPRESSION VON 5HTR2C UND/ODER ADARS ALS MARKER DER VERÄNDERUNG DES MECHANISMUS DES 5HTR2C-MRNA-EDITINGS UND DEREN ANWENDUNGEN
ÉCHANTILLON DE TISSU PÉRIPHÉRIQUE CONTENANT DES CELLULES EXPRIMANT 5HTR2C ET/OU LES ADAR, UTILISÉES COMME MARQUEURS DE L'ALTÉRATION DU MÉCANISME D'ÉDITION DE L'ARNM 5HTR2C ET SES APPLICATIONS

(30) Priority: 13.06.2007 US 943685 P; 24.01.2008 US 23239 P
(43) Date of publication of application: 24.01.2018
(62) Divisional of application: 08761038.2
(73) Proprietor: ALCEDIAG, 13790 Peynier (FR)
(72) Inventor: PUJOL, Jean-François, 34270 st Mathieu de Tréviers (FR); WEISSMANN, Dinah, 34270 Mathieu de Treviers (FR); VINCENT, Laurent, 91620 La Ville du Bois (FR); CAVAREC, Laurent, 94300 Vincennes (FR); MANN, John, New York, NY 10471 (US)
(74) Representative: Touroude, Magali Linda

(56) References cited:
- WO-A-2005/087949
- WO-A-2006/032859
- WO-A-2006/134128
- US-A- 5 763 174
- YANG WEIDONG ET AL: "Altered RNA editing of serotonin 5-HT2C receptor induced by interferon: implications for depression associated with cytokine therapy.", BRAIN RESEARCH. MOLECULAR BRAIN RESEARCH 29 APR 2004, vol. 124, no. 1, 29 April 2004 (2004-04-29), pages 70-78, XP002498423, ISSN: 0169-328X
- OLGA A SERGEEVA ET AL: "Editing of AMPA and Serotonin 2C Receptors in Individual Central Neurons, Controlling Wakefulness", CELLULAR AND MOLECULAR NEUROBIOLOGY, KLUWER ACADEMIC PUBLISHERS-PLENUM PUBLISHERS, NE, vol. 27, no. 5, 7 June 2007 (2007-06-07), pages 669-680, XP019524896, ISSN: 1573-6830
- SCHMAUSS C: "SEROTONIN 2C RECEPTORS: SUICIDE, SEROTONIN, AND RUANWAY RNA EDITING", NEUROSCIENTIST, BALTIMORE, MD, US, vol. 9, no. 4, 1 August 2003 (2003-08-01), pages 237-242, XP009033018, ISSN: 1073-8584
- SHIN KWAK ET AL: "Deficient RNA editing of GluR2 and neuronal death in amyotropic lateral sclerosis", JOURNAL OF MOLECULAR MEDICINE, SPRINGER, BERLIN, DE, vol. 83, no. 2, 1 February 2005 (2005-02-01), pages 110-120, XP019320322, ISSN: 1432-1440
- NISWENDER COLLEEN M ET AL: "RNA editing of the human serotonin 5-HT2C receptor: Alterations in suicide and implications for serotonergic pharmacotherapy", NEUROPSYCHOPHARMACOLOGY, ELSEVIER SCIENCE PUBLISHING, NEW YORK, NY, US, vol. 24, no. 5, 1 May 2001 (2001-05-01), pages 478-491, XP002288022, ISSN: 0893-133X
- S DRACHEVA ET AL: "Increased serotonin 2C receptor mRNA editing: a possible risk factor for suicide", MOLECULAR PSYCHIATRY, vol. 13, no. 11, 11 September 2007 (2007-09-11), pages 1001-1010, XP055592628, GB ISSN: 1359-4184, DOI: 10.1038/sj.mp.4002081

## Description

The present invention relates to an in vitro method for predicting a pathology related to an alteration of the mechanism of the mRNA editing of ADAR dependent A to I mRNA editing, particularly the serotonin 2C receptor (5HTR2C), in a patient from a peripherical tissue sample containing cells expressing said mRNA, such as the 5HTR2C mRNA, and/or adenosine deaminases acting on RNA (ADARs), such as skin and/or blood tissue sample. The present invention further comprises a method for identifying if an agent is capable of in vivo modifying the editing of the 5HTR2C mRNA in brain tissue or to control the efficiency of a drug intended to prevent or to treat a pathology related to an alteration of the mechanism of the 5HTR2C mRNA editing brain tissue, these methods comprising the implementation of said peripherical tissue markers. In a particular aspect, the present invention relates to such methods wherein the 5HTR2C mRNA editing rate or profile, when it is necessary, is determined by a single strand conformation polymorphism (SSCP) method after amplification by a PCR, preferably by a nested PCR, of the specific mRNA fragment containing the edition sites, making it possible, under given analytical conditions, to obtain the editing rate and/or profile of this edited 5HTR2C mRNA from said peripherical tissue. Finally the invention relates to particular nucleic acid primers implemented in said nested PCR.

Genetic association studies, knockout mice and postmortem analysis have suggested the implication of the serotonin 2C receptor (5HTR2C) in neuropsychiatric disorders. Firstly, a functional allelic polymorphism (Cys23Ser) of 5HTR2C is associated with depression and bipolar disorder (Lerer et al., 2001, Mol. Psychiatry, 6:579-585). Secondly, mice lacking the 5HT2C serotonin receptor exhibit spontaneous convulsions, cognitive impairment and abnormal control of feeding behavior (Tecott et al., 1995, Nature, 374:542-546). These animals are also hyper responsive to repeated stress (Chougreen et al., 2003, Physiol. Behav., 79:217-226). Thirdly, in postmortem brains of patients affected by bipolar disorder or schizophrenia, the RNA expression of the 5-HT2C serotonin receptor is down-regulated (Iwamoto et al., 2004, MoI. Psychiatry, 9:406-416; Castensson et al., 2003, Biol. Psychiatry, 54:1212-1221).

RNA editing of 5HTR2C is also thought to be involved in the pathophysiology of mental disorders and the action of antidepressants (Seeburg, 2002, Neuron, 35:17-20). Five adenosine residues are edited in a region coding for the second intracellular loop of the 5HTR2C and can lead to amino-acid substitutions at three different positions of the receptor sequence. The combinational substitution of these amino residues generates up to 24 different 5HTR2C protein isoforms with different G-coupling efficiencies (Price et al, 2001, J. Biol. Chem., 276:44663-44668). In mice, when compared with C57BL/6 and 129sv inbred strains, the BALB/c strain exhibits a different basal forebrain neocortical 5HTR2C pre-mRNA editing pattern that may underlie their difference in stress reactivity. Moreover, the BALB/c mice exhibit stress-induced changes in 5HTR2C pre-mRNA editing resembling those detected in brains of depressed suicide victims (Englander et al., 2005, J. Neurosci., 25:648-651). Actually, in postmortem brains, altered RNA editing of 5HTR2C has been reported in patients with schizophrenia, depression and those who committed suicide (Niswender et al., 2001, Neuropsychopharmacology, 24:478-491; Sodhi et al., 2001, Mol. Psychiatry, 6:373-379; Gurevich et al., 2002, Neuron, 34:349-356). Additionally interferon is used in hepatitis C treatment but symptoms of depression often appear as a side effect of this molecule in patients and Yang et al. have demonstrated that this molecule strongly alters the editing of 5HTR2C (see ref. in Tohda et al., 2006, J. Pharmacol. Sci., 100, 427-432).

Previous studies have shown that the 5HTR2C is mainly expressed in the brain, particularly in choroid plexus, prefrontal cortex, limbic areas, basal ganglia and hypothalamus (Tohda et al., 1996, Neurosci. Res., 24:189-193; Julius et al., 1988, 241 :558-564; Pasqualetti et al., 1999, Neuroscience, 92:601-611). This brain specific pattern of expression restricts investigations of potential links existing between 5HTR2C RNA editing and psychiatric condition to post-mortem studies. In search of more easily available tissues, possibly mirroring the editing status of HTR2C in CNS, and allowing quantitative analysis in patients with different psychiatric states, Marazziti and collaborators have detected the presence of 5HTR2C mRNAs in resting lymphocytes (Marazziti et al., 2001, Neuropsychobiology, 43:123-126). Unfortunately in these cells the level of expression of the 5HTR2C, as revealed by RT-PCR / Southern-blotting, is too low for further quantitative RNA editing analysis.

Recently, Slominski and co-workers have shown that human skin and cultured skin-derived cells have the capability to transform L-tryptophan to serotonin and to metabolize serotonin to N-acetyl-serotonin and melatonin (Slominski et al, 2002, FASEB J., 16:896-898). They have further tested by nested RT-PCR the expression of genes encoding the receptors of this cutaneous serotoninergic/melatoninergic metabolic pathway. Whole human skin and normal and pathological cultured skin cells predominantly express genes encoding the 5-HT2B and 5HT7 iso forms of the serotonin receptor. The expression of other serotonin receptors iso forms is less prevalent and 5HTR2C rarely detected (Slominski et al., 2003, J. Cell. Phys., 196:144-153).

The following documents can also be cited :
WO2006032859 (A2) (Bruinvels Anne T et al.) discloses methods for determining whether a patient diagnosed with schizophrenia, or suffering from suicidal behaviour or from mild cognitive impairment, is suitable for treatment with a 5-HT2C receptor antagonist, and associated uses of 5-HT2C receptor antagonists.

WO2006134128 (A2) (Pohlner Johannes et al.) describes a dysregulation of the ADARB2 gene and the protein products thereof in Alzheimer's disease patients. It is discloses methods for diagnosing and prognosticating Alzheimer's disease in a subject, and for determining whether a subject is at increased risk of developing Alzheimer's disease.

WO2005087949 (A1) (Levanon Erez et al.) discloses a method for detecting an RNA editing site, and methods of use and assays thereof. It is describes that RNA editing by members of the double-stranded RNA-specific ADAR family leads to site-specific conversion of adenosine to inosine (A-to-I) in the precursor messenger RNAs1. Editing by ADARs is believed to occur in all metazoa, and is essential for mammalian development2 and nd that quantitation of inosine in total RNA suggests that editing affects a much larger fraction of the mammalian transcriptome.

Weidong Yang et al ; (Brain Res Mol Brain Res. 2004 Apr 29;124(1):70-8., discloses that members of the ADAR gene family are involved in one type of RNA editing that converts adenosine residues to inosine. It is also disclosed that RNA editing may play a role in several pharmacological and behavioral processes where the serotonergic plasticity is mediated through 5-HT(2C)R. The authors examined the effects of IFN-alpha on RNA editing in human glioblastoma cell lines, which express 5-HT(2C)R mRNAs. supporting the hypothesis that 5-HT(2C)R mRNA editing has causative relevance in the pathophysiology of depression associated with cytokine therapy.

Olga A Sergeeva et al ; (Cell Mol Neurobiol. 2007 Aug;27(5):669-80.) discloses that pre-mRNA editing of serotonin 2C (5-HT2c) and glutamate (Glu) receptors (R) influences higher brain functions and pathological states such as epilepsy, amyotrophic lateral sclerosis, and depression.. The authors disclose that the 5-HT2cR mRNA was always edited at A, in the majority of cells at B sites and variably edited at E, C and D sites. A negative correlation was found between editing of C and D sites. They indicates that further factors/enzymes besides ADAR must control or influence 5-HT2cR and that these factors vary between individuals and could be predictors of psychiatric disease.

Claudia Schmauss (Neuroscientist. 2003 Oct;9(5):419) discloses that transcripts of the gene encoding the serotonin 2C receptor are modified by RNA editing, and that studies on mice revealed that 5-HT2C pre-mRNA editing is regulated in a serotonin-dependent manner, and postmortem studies on brain tissues of patients with schizophrenia and major depression found distinct site-specific alterations of this editing in the prefrontal cortex. The author describes that most complex alterations in 5-HT2C pre-mRNA editing were found in brains of depressed suicide victims. In these brains, 5-HT2C receptor isoforms with reduced function are expressed at significantly increased levels, suggesting that the regulation of editing by synaptic serotonin is defective.

Shin Kwak e tal. (J Mol Med (Berl). 2005 Feb;83(2):110-20), dicloses that one plausible hypothesis for selective neuronal death in sporadic amyotropic lateral sclerosis (ALS) is excitotoxicity mediated by alpha-amino-3-hydroxy-5-methyl-4-isoxazolepropionate (AMPA) receptors, which are a subtype of ionotropic glutamate receptors. whose properties (GluR2) are generated posttranscriptionally by RNA editing at the Q/R site The authors indicates that it is likely that the molecular mechanism underlying the deficiency in RNA editing is a reduction in the activity of ADAR2, and that the restoration of this enzyme activity in ALS motor neurons may open the novel strategy for specific ALS therapy.

Members of the ADAR (adenosine deaminases acting on RNA) gene family are involved in one type of RNA editing that convert adenosine residues to inosine. The process of RNA editing is a widespread phenomenon in eukaryotes that leads to posttranscriptional base changes in mRNA. In mammals, a growing number of genes have been identified that undergo a type of RNA editing that is characterized by site-selective adenosine-to-inosine modification.

Among A-to-I editing substrates are the brain-specific transcripts coding for the glutamate receptors AMPA type (such as GluR2 and GluR4) and G-protein-coupled serotonin receptors (such as 5HTR2C). In GIuR subunit B (GIuR-B), a single editing position (the Q/R-site) controls the Ca2+-permeability of the ion channel, whereas another position (the R/G-site) regulates the desensitization kinetics of the receptor. This property of AMPA receptors is critical for normal brain function. Because of the importance of accurate RNA editing for normal brain function, the deregulation of editing activity may influence the progression of pathophysiological processes, such as neurodegenerative diseases or tumorigenesis (epilepsie cognitif, tumors, sleep waking, mood disorders eating (Maas S et al. (1996) J. Biol. Chem 271, 12221-26; Sergeeva OA et al. (2007) Cell MoI Neurobiol. 27:669-680.

Another ADAR A-to-I editing substrate which has been identified at the level of T cells as an isoenzyme of phosphodiesterases, is the phospodiesterase 8Al. 6 to 7 sites of editing have been identified and could be modulated in pathological state ( lupus erythematosus) and after drug action (interferon alpha).

It is important to note that this isoenzyme is present in brain (OrIo wski RJ et al, 8A1 gene transcripts of systemic lupus erythematosus T lymphocytes. Immunology 2008 in press; Wang P et al., phosphodiesterase 8 A (PDEA8) splice variants: cloning, gene organization and tissue distribution. (2001) Gene 280 183-194).

Among these ADAR brain-specific substrates, the A-to-I editing of 5HTR2C mRNA leads to replacement of three amino acid residues located within the intracellular loop II domain, resulting in dramatic alterations in G-protein coupling functions of the receptor (Yang W et al., Brain Res MoI Brain Res. 2004, 124(l):70-78). Four A-to-I RNA editing enzymes, termed ADAR1, ADAR2, ADAR3 and ADAT1, have been cloned from mammals. ADAR1 iso forms and ADAR2 are widely expressed in a variety of cells and tissues with the highest expression in the brain and spleen and are the essential ADARs involved in 5HTR2C mRNA editing (ADAR 3 was identified solely in the brain and its deaminase activity has not yet been established and ADATI targets tRNA). 5HTR2C mRNA is edited at five closely spaced adenosine residues (termed A, B, C, D and E editing sites) allowing the generation of 32 different mRNA variants and 24 different protein iso forms of the receptor ranging from the unedited Ilel56-Asnl58-Ilel6O (INI) isoform to the fully edited Vall56-Glyl58-Vall60 (VGV) isoform. It is known that ADAR1 alone is involved in the A and B editing sites, both ADAR1 and ADAR2 in the E and C editing sites, and ADAR2 alone in the D editing site (Dracheva et al., Molecular Psychiatry, 2007, 1-10).

It is known that interferon-alpha (IFN-alpha) often causes severe depression in patients treated for chronic viral hepatitis and certain malignancies. The effects of IFN-alpha on RNA editing in human glioblastoma cell lines has been observed (Yang et al., Beyond the Identification of Transcribed Sequences: Functional, Evolutionary and Expression Analysis, 12th International Workshop, October 25-28, 2002, Washington, DC, Intracellular Trafficking of A Few Inflammation-Inducible ADAR1 Isoform). It has been also observed, in vivo in the Balb/c Mouse, that the administration of interferon alpha, known to be a powerful activator of the expression of ADAR1 150 in vitro in human glioblastoma cells lines (Yang W. et al., 2004), induces also subsequent changes in the editing profile of the 5HTR2C in the dorsal prefrontal cortex.

In order to allow rapid and validated predictive parameters of general modifications of the editing process, it remains desirable:
- to provide a method allowing to extrapolate alterations of the editing rate or profile of these ADAR substrates, particularly the glutamate receptors AMPA type or the 5HTR2C mRNA, in human brain tissue from a biological sample which can be obtained easily from the patient to be tested, and wherein, it will be preferred that the editing rate or profile of these ADAR substrates determined in this biological sample could be correlated with this obtained from brain biological sample; and/or
- to identify a marker present in a biological sample which can be obtained easily from the patient to be tested, wherein the qualitative and/or quantitative analysis of said marker in said sample can be correlated to an alteration of the editing rate or profile of these ADAR substrates in brain tissue, such biological sample and associated marker could be used as a reporter of the receptor editing observed in CNS (central nervous system).

This is precisely the subject of the present invention.

The present invention describes the use of or a method implementing a single biological sample or two different biological samples selected from the group of biological sample consisting of peripherical tissues containing cells for evaluating the pathological alteration of a mRNA editing in the brain and wherein said mRNA editing is an ADAR dependent A to I mRNA editing.

The present invention also describes the use or to a method implementing as a single or an associated reporter sample for evaluating the pathological alteration of said mRNA editing in the brain, of:
- a first peripherical tissue containing cells, such as a skin sample from a mammal; or/and
- a second peripherical tissue containing cells, such as a blood sample from a mammal.

In a preferred embodiment, said edited mRNA whose editing is an ADAR dependent A to I mRNA editing, is a mRNA selected from the group consisting of the mRNA coding for a glutamate receptor AMPA type, for a G-protein-coupled serotonin receptor and for the PDEA8.

In a preferred embodiment, the evaluation of the pathological alteration of the mRNA editing in the brain is determining by:
- the editing rate(s) or profile of the edited forms of said mRNA in said peripherical biological sample; and/or
- the nature or/and the quantity of the ADARs expressed in said peripherical biological sample.

In a more preferred embodiment, said mRNA having an ADAR dependent A to I mRNA editing is the 5HTR2C mRNA.

The inventors have found that the measure of changes in ADARs expression at the periphery (such in blood) could predict important alteration of editing in the brain.

The inventors have demonstrated for example that the determination of the 5HTR2C mRNA editing and/or the determination of the ADARs activities expressed in peripherical tissue containing cells expressing the 5HTR2C and/or ADARs, such as skin and/or blood tissue sample, can be used as reporter markers of the alteration of the mechanism of the 5HTR2C mRNA editing in the brain tissue and thus, for evaluating the pathological alteration of the multistep-metabolic pathway of the serotinergic system expressed in the CNS .

The inventors have for example demonstrated that the essential ADARs responsible of the editing of the mammal 5HTR2C, which are the two ADAR1 iso forms (named hereinafter "ADAR1 -150" and "ADAR1-110" for ADAR1 -150 kD protein and ADAR1-110 kD protein) and ADAR2, are all expressed in sufficient quantity in said peripherical tissue containing cells, particularly in blood sample white cells, in order to be qualitatively and/or quantitatively analysed and to use this peripherical tissue containing cells and the ADARs as a reporter sample and marker for evaluating the pathological alteration of the multistep-metabolic pathway of the serotinergic system expressed in the CNS.

They have also demonstrated that contrary to what it has been indicated in the prior art for skin sample (Slominski et al, 2003, J. Cell Phy., 196, 144-153), certain peripherical tissue containing cells, such as skin sample cells, express sufficient 5HTR2C mRNA to be detected and analysed to evaluate the editing rate or profile of the 5HTR2C mRNA, and optionally, the nature and/or the quantity of the ADARs contained.

Consequently, - the determination of an altered or normal expression (in nature and/or in quantity) of the ADARs enzymes, particularly ADAR1 iso forms (particularly the "ADAR1 -150" and "ADAR1-110") and ADAR2, in peripherical tissue containing cells expressing these ADARs, such as in skin and/or blood tissue sample containing cells; and/or - the determination of the 5HTR2C mRNA editing rate or profile of the edited forms of the 5HTR2C mRNA in peripherical tissue containing cells expressing the 5HTR2C mRNA, such as in skin and/or blood tissue sample containing cells,can be used as reporter markers of the alteration of the mechanism of the 5HTR2C mRNA editing in the brain tissue and thus, for evaluating the pathological alteration of the multistep-metabolic pathway of the serotinergic system expressed in the CNS of a patient.

Finally, the determination of an altered or normal expression of the ADARs enzymes, alone or in association with the determination of the 5HTR2C mRNA editing rate or profile of the edited forms of the 5HTR2C mRNA, in one or more peripherical tissue samples containing cells, such as in skin and/or blood tissue sample containing cells, can be used as reporter sample(s) and markers:
- to identify in vitro whether a patient presents a pathology or is at risk to develop a pathology related to an alteration of the mechanism of the mRNA editing of the serotonin 2C receptor (5HTR2C);
- to determine in vitro whether a pathology exhibited by a patient is related to an alteration of the mechanism of the mRNA editing of the 5HTR2C;
- to select a compound capable of modulating the 5HTR2C mRNA editing in the brain tissue, preferably compound able to restore the normal 5HTR2C mRNA editing in the brain tissue of a patient in need thereof; or
- to determine in vitro in a mammal the efficiency of a drug used for the prevention or for the treatment of a pathology related to an alteration of the mechanism of the mRNA editing of the 5HTR2C.

By "peripherical tissue" containing cells, it is intended to designate herein tissue other than brain tissue and which is preferably easy to collect, such as in general biopsy of organ or tissue easy to collect, skin sample, whole blood sample, urine sample, saliva sample, internal cheek tissue sample, vagina or internal cheek exfoliative cytology or smear. Skin and/or blood sample containing cells are the preferred peripherical tissue sample implementing in the present invention.

By association of these two markers (ADARs and 5HTR2C mRNA editing), it is intended that the ADARs expression can be analysed in the same type of cells as the cells used for the determination of the 5HTR2C mRNA editing, for example in skin cells sample), or that the ADARs expression can be analysed in one type of cells, for example blood cells sample, and the determination of the 5HTR2C mRNA editing is carried out in another type of cells, for example in skin cells sample.

Each marker can be used also alone whether the determination of this marker is sufficient to correlate its expression in a peripherical tissue samples containing cells, such as in skin and/or blood tissue sample containing cells, with the editing rate or profile of the 5HTR2C mRNA in brain tissue.

For example, the determination of an altered or normal expression of the ADARs in blood tissue sample cells, such as white cells, can be used alone as a reporter marker of the alteration of the mechanism of the 5HTR2C mRNA editing in the brain tissue whether the correlation obtained is sufficient with single marker.

For another example, the determination of the 5HTR2C mRNA editing rate or profile of the edited forms of the 5HTR2C mRNA or the determination of an altered or normal expression of the ADARs in skin tissue sample containing cells expressing the 5HTR2C mRNA and ADARs, can be also alone as a reporter marker of the alteration of the mechanism of the 5HTR2C mRNA editing in the brain whether the correlation obtained is sufficient with the single marker used.

Concerning the method to select compound capable of modulating the 5HTR2C mRNA editing, the cells of the peripherical tissue expressing the 5HTR2C mRNA and/or ADARs for evaluating and selected such compounds can be cells lines or recombinant cell lines wherein the expression of the 5HTR2C mRNA and/or ADARs have been altered in order, for example, to mimic pathological expression of this 5HTR2C mRNA and/or ADARs. Skin or blood recombinant cells or cell lines can be particularly used for this aspect.

The present invention comprises a method implementing a single biological sample selected from the group of biological sample consisting of peripherical tissues containing cells for evaluating the pathological alteration of a mRNA editing in the brain and wherein said mRNA editing is an ADAR dependent A to I mRNA editing;
- wherein said peripherical tissue containing cells is selected from the group consisting whole blood sample or blood sample containing buffy coat;
- wherein said edited mRNA is 5HTR2C mRNA;
- evaluating the pathological alteration of the 5HTR2C mRNA editing in the brain by determining the ADAR expression products ADAR1, isoforms 150 and/or 110, and the ADAR2 gene expression products expressed in said sample.

The present invention also comprises a method for identifying in vitro whether a patient presents a pathology or is at risk to develop a pathology related to an alteration of the mechanism of the mRNA editing of the 5HTR2C, wherein this method comprising the following steps of:
a) determining the ADAR expression products ADAR1, isoforms 150 and/or 110, and the ADAR2 gene expression products in a biological sample containing blood cells obtained from the patient to be tested;
b) identifying whether said patient presents or is at risk to develop such a pathology by comparing the quantity of the ADARs expressed in said sample with expressed ADARs profil obtained for normal patients or for patients exhibiting pathologies related to an alteration of the mechanism of this mRNA editing.

In a preferred embodiment, said pathology is selected from the group consisting of mental disorders, schizophrenia, depression, depressed suicide or abnormal feeding behaviour.

In a preferred embodiment, said pathology is selected from the group consisting of depression, and depressed suicide behaviour.

The present invention also comprises a method for determining in vitro whether a pathology exhibited by a patient is related to an alteration of the mechanism of the mRNA editing of the 5HTR2C, wherein this method comprising the following steps of:
a) determining the quantity of the ADARs expressed in a biological sample containing blood cells obtained from the patient;
b) identifying whether said patient presents or is at risk to develop such a pathology by comparing the quantity of the ADARs expressed in said sample with expressed ADARs profil obtained from normal patients or from patients exhibiting pathologies known to be not related to an alteration of the mechanism of this mRNA editing.

The present invention also comprises a method for identifying in vitro an agent that modulates in vivo the editing of the 5HTR2C mRNA in a mammal, comprising the following step of:
- comparing the ADAR expression products ADAR1, isoforms 150 and/or 110, and the ADAR2 gene expression products in a biological sample containing blood cells obtained from the mammal after treatment with said agent with the ADAR expression products obtained from control blood sample obtained from said mammal.

The present invention also comprises a method for identifying in vitro an agent that modulates the editing of the 5HTR2C mRNA in a mammal, comprising the following steps of:
a) contacting a biological sample containing mammalian blood cells line in the presence of a candidate modulator of said 5HTR2C mRNA editing; and
b) determining the effects of said modulator on the ADAR expression products ADAR1, isoforms 150 and/or 110, and the ADAR2 gene expression products expressed in said sample of blood cells, by comparing the quantity of the ADARs expressed obtained from the biological sample in step a) with the quantity of the ADARs expressed obtained from control tissue sample of bloods cells of said mammal.

The present invention also comprises a method method for determining in vitro in a patient the efficiency of a drug used for the prevention or for the treatment of a pathology related to an alteration of the mechanism of the mRNA editing of the 5HTR2C, comprising the following steps of:
a) determining the ADAR expression products ADAR1, isoforms 150 and/or 110, and the ADAR2 gene expression products in a blood sample containing blood cells obtained from the patient; and
b) determining the efficiency of said drug by comparing the ADAR expression products ADAR1, isoforms 150 and/or 110, and the ADAR2 gene expression products obtained in step a) with the ADAR expression products obtained from the patient during or/and after the treatment with said drug,
wherein a modulation of the ADAR expression products expressed resulting to ADAR expression products expressed close or equal to this observed for normal patients being significant of the efficiency of the treatment.

The present invention also comprises a method for determining if a patient responds or does not respond to a treatment of a pathology resulting or provoking by the alteration of the mechanism of the mRNA editing of the 5HTR2C, comprising the following steps of:
a) determining the ADAR expression products ADAR1, isoforms 150 and/or 110, and the ADAR2 gene expression products in a blood sample containing blood cells obtained from the patient before the beginning of the treatment;
b) determining if the patient responds or not responds to the treatment by observing the modification of the ADAR expression products after a period of treatment by comparing with the quantity of the ADARs expression products obtained in step a) before the beginning of the treatment.

Such a method allows to avoid to extend needlessly the period of treatment with a drug if it can be thus possible to determine rapidly that the patient does not respond to that drug, or to continue the treatment if the patient responds to that drug.

Editing is the mechanism by which information contained in the gene is modified after transcription. The general term "mRNA editing" includes the modification of the sequence of these mRNAs which results in a change, in terms of nature or number, in the amino acids incorporated into the protein during translation, it no longer being possible for the sequence of the protein to be deduced from that of the gene which directs its synthesis. The pre-messenger RNA of 5HTR2C can undergo a specific enzymatic modification of certain adenosines (A), in the portion of what will become the definitive mRNA which directs the incorporation of the amino acids located in the second intracellular loop of 5HTR2C. In fact, the distal part of the fifth exon and the proximal part of the fifth intron of the primary transcript are capable of forming a stem-loop structure potentially recognized by two enzymes, ADARl and ADAR2 (double-stranded RNA-dependent adenosine deaminase), which make it possible to edit the premessenger RNA before it is spliced. This editing is produced by deamination of As, which are then converted to inosine (I). Once the splicing has been completed, the part of the mRNA which contained the As which underwent the editing now contains Is. When the 5HTR2C mRNA is translated, it is thought that the Is are read as Gs. In fact, during in vitro synthesis of the cDNA from the 5HTR2C mRNA that underwent the deamination of As to Is, the reverse transcriptase incorporates dCs opposite the Is, instead of dTs which should normally have been incorporated opposite the As. Consequently, during the synthesis of the second strand which results in the formation of the double-stranded cDNA, a dG is introduced opposite each dC incorporated into the first strand. Sequencing of the double-stranded cDNA thus obtained makes it possible to observe the replacement of the dAs with dGs, due to the initial deamination of the As to Is in the mRNA which underwent the editing. Consequently, the editing of the mRNA results in a modification of the meaning of the codons in which the As are replaced with Is, which are therefore thought to be read as Gs (more specifically, with regard to the editing of human 5HTR2C, see Fitzgerald et al, Neuropsychopharmacology, 1999, 21(2S), 82S-90S).

So, the determination of an alteration of the mechanism of the mRNA editing of the 5HTR2C by the control of the editing rate in skin sample is also significant of an alteration of the multistep-metabolic pathway of the serotoninergic system expressed in the skin and which could be used as a reporter of the serotoninergic system expressed in brain.

The term "edited RNA" is intended to denote, in the present description, any RNA sequence in which at least one adenosine has been deaminated to inosine by an adenosine deaminase.

By "editing rate", it is intended to designate the percentage of each of the edited and unedited forms of the mRNA which may comprise at least one editing site, relative to the total amount of the edited or unedited mRNA forms present in said same sample.

| Editing sites | | A B EC D | |
|---|---|---|---|
| 1 | 5HTR2C-0 | ATACGTAATCCTATT | SEQ ID NO. 1 |
| | I-N-I | | |
| 2 | 5HTR2C-A | ITACGTAATCCTATT | SEQ ID NO. 2 |
| | V-N-I | | |
| 3 | 5HTR2C-B | ATICGTAATCCTATT | SEQ ID NO. 3 |
| | M-N-I | | SEQ ID NO. 4 |
| 4 | 5HTR2C-C | ATACGTAITCCTATT | |
| | I-S-I | | |
| 5 | 5HTR2C-D | ATACGTAATCCTITT | SEQ ID NO. 5 |
| | I-N-V | | |
| 6 | 5HTR2C-E | ATACGTIATCCTATT | SEQ ID NO. 6 |
| | I-D-I | | |
| 7 | 5HTR2C-AB | ITICGTAATCCTATT | SEQ ID NO. 7 |
| | V-N-I | | |
| 8 | 5HTR2C-AC | ITACGTAITCCTATT | SEQ ID NO. 8 |
| | V-S-I | | |
| 9 | 5HTR2C-AD | ITACGTAATCCTITT | SEQ ID NO. 9 |
| | V-N-V | | |
| 10 | 5HTR2C-AE | ITACGTIATCCTATT | SEQ ID NO. 10 |
| | V-D-I | | |
| 11 | 5HTR2C-BC | ATICGTAITCCTATT | SEQ ID NO. 11 |
| | M-S-I | | |
| 12 | 5HTR2C-BD | ATICGTAATCCTITT | SEQ ID NO. 12 |
| | M-N-V | | |
| 13 | 5HTR2C -BE | ATICGTIATCCTATT | SEQ ID No. 13 |
| | M-D-I | | |
| 14 | 5HTR2C -CD | ATACGTAITCCTITT | SEQ ID No. 14 |
| | I-S-V | | |
| 15 | 5HTR2C -CE | ATACGTIITCCTATT | SEQ ID No. 15 |
| | I-G-I | | |
| 16 | 5HTR2C -DE | ATACGTIATCCTITT | SEQ ID No. 16 |
| | I-D-V | | |
| 17 | 5HTR2C-ABC | ITICGTAITCCTATT | SEQ ID No. 17 |
| | V-S-I | | |
| 18 | 5HTR2C -ABD | ITICGTAATCCTITT | SEQ ID No. 18 |
| | V-N-V | | |
| 19 | 5HTR2C -ABE | ITICGTIATCCTATT | SEQ ID No. 19 |
| | V-D-I | | |
| 20 | 5HTR2C -ACD | ITACGTAITCCTITT | SEQ ID No. 20 |
| | V-S-V | | |
| 21 | 5HTR2C -ACE | ITACGTIITCCTATT | SEQ ID No. 21 |
| | V-G-I | | |
| 22 | 5HTR2C -ADE | ITACGTIATCCTITT | SEQ ID No. 22 |
| | V-D-V | | |
| 23 | 5HTR2C -BCD | ATICGTAITCCTITT | SEQ ID No. 23 |
| | M-S-V | | |
| 24 | 5HTR2C -BCE | ATICGTIITCCTATT | SEQ ID No. 24 |
| | M-G-I | | |
| 25 | 5HTR2C -BDE | ATICGTIATCCTITT | SEQ ID No. 25 |
| | M-D-V | | |
| 26 | 5HTR2C -CDE | ATACGTIITCCTITT | SEQ ID No. 26 |
| | I-G-V | | |
| 27 | 5HTR2C -ABCD | ITICGTAITCCTITT | SEQ ID No. 27 |
| | V-S-V | | |
| 28 | 5HTR2C -ABCE | ITICGTIITCCTATT | SEQ ID No. 28 |
| | V-G-I 2 | | |
| 9 | 5HTR2C-ABDE | ITICGTIATCCTITT | SEQ ID No. 29 |
| | V-D-V | | |
| 30 | 5HTR2C-ACDE | ITACGTIITCCTITT | SEQ ID No. 30 |
| | V-G-V | | |
| 31 | 5HTR2C-BCDE | ATICGTIITCCTITT | SEQ ID No. 31 |
| | M-G-V | | |
| 32 | 5HTR2C-ABCDE | ITICGTIITCCTITT | SEQ ID No. 32 |
| | V-G-V | | |

| | | | |
|---|---|---|---|
| * The editing site " E " is also named " C " | | | |

In a preferred embodiment of the methods according to the invention, the skin cells are selected from the group consisted of keratinocytes, melanocytes, fibroblasts, Langerhans cells and Merkels cells, and the skin tissue is selected from the group consisted of epidermis and dermis.

It is also described a kit for the determination of a mammal 5HTR2C mRNA editing rate or profile, preferably in human, in rat or in mouse, more preferably in human, wherein said kit contains a nucleic acid according to the invention. In a preferred embodiment of the methods of the present the cells which are selected for determining alone or in association the nature and/or the quantity of the expressed ADARs are blood white cells or leucocytes or originated from the buffy coat of a whole blood sample obtained after centrifugation.

In a preferred embodiment, in step b) of the methods of the present invention, the ADAR expression products are ADAR1, iso forms 150 and/or 110, and the ADAR2 gene expression products, preferably the expression products of the mouse, rat or human gene encoding the ADARl, isoforms 150-kD and/or 110-kD protein, and the ADAR2 protein. Human is the most preferred.

The ADAR1 mRNA nucleic sequence encoding the protein isoforms 150 kD and 110 kD, and the ADAR2 mRNA nucleic sequence encoding the ADAR2 protein, and its amino acid sequence are well known from the skilled person for the human, mouse or rat.

For example, the following sequences depicted in Genbank under the accession number can be particularly cited:
for ADAR1 :
   - for Human: NM_00l111.3; NM_001025107.1,
   - for Mouse: NM_019655.2; NM_001038587.2,
for ADAR2:
   - for Human: NM_001l12.2; NM_015833.2; NM_015834.2 and NM_001033049.1,
   - for Mouse :NM_130895.2; NM_00102483.7; NM_001024838.1; NM_001024840.1 and NM_001024839.1.

In a more preferred embodiment, in step b), the ADAR expression products are the ADAR mRNAs.

In a prefrred embodiement, in the method of the present invention, in step b), the determination of the ADAR mRNA is carried out by a method which comprises the following steps:
A) extraction of the total RNAs of said blood sample cells, followed, where appropriate, by purification of the mRNAs;
B) reverse transcription of the RNAs extracted in step A) via an oligo dT primer; and
C) PCR amplification of the cDNAs obtained in step B) using at least a pair of primers specific for each of the ADAR mRNA to be quantified and/or qualitatively analysed.

In a preferred embodiment, the pair of primers specific for the ADAR mRNA PCR amplification are selected from the group consisting of:
- for human ADAR1 -150 isoform mRNA amplification:
   EXlA 34p Forward: 5 '-GCCTCGCGGGCGCAATGAATCC-S ' (SEQ ID NO. 41), EX2
   578m Reverse: 5 '-CTTGCCCTTCTTTGCCAGGGAG-3 ' (SEQ ID No. 42);
- for human ADARl-110 isoform mRNA amplification:
   EXlB 534p Forward: 5 '-CGAGCCATCATGGAGATGCCCTCC-S ' (SEQ ID NO. 43),
   EX2 804m Reverse: 5 '-CATAGCTGCATCCTGCTTGGCCAC-S ' (SEQ ID NO. 44);
- for human ADAR2 mRNA amplification:
   ADAR2 1274p Forward: 5 '-GCTGCGCAGTCTGCCCTGGCCGC-S ' (SEQ ID No. 45),
   ADAR2 1486m Reverse: 5 '-GTCATGACGACTCCAGCCAGCAC-S ' (SEQ ID No. 46);
- for mouse ADAR1 -150 isoform mRNA amplification:
   EX1A 19p Forward: 5 '-GTCTCAAGGGTTCAGGGGACCC-S ' (SEQ ID NO. 47), EX2
   646m Reverse: 5 '-CTCCTCTAGGGAATTCCTGGATAC-S ' (SEQ ID NO. 48);
- for mouse ADARl-110 isoform mRNA amplification:EXlB 72p Forward: 5 '-TCACGAGTGGGCAGCGTCCGAGG-S ' (SEQ ID NO. 49), EX2 646m Reverse: 5 '-CTCCTCTAGGGAATTCCTGGATAC-S ' (SEQ ID NO. 48); and
   - for mouseADAR2 mRNA amplification:
      EX7 128 Ip Forward: 5 '-GCTGCACAGTCTGCCTTGGCTAC-S ' (SEQ ID NO. 50), EX9 1622m Reverse: 5 '-GCATAAAGAAACCTGAGCAGGGAC-S ' (SEQ ID NO. 51);

In a prefrerred embodiement, in the the method according to the present invention, in step b), the ADAR expression products are the ADAR proteins.

Thus, in a preferred embodiment of this method, the determination of the ADAR proteins is carried out by a method which comprises the following steps:
A) optionally, the extraction of the total proteins contained in said blood sample cells, followed, where appropriate, by a step of proteins purification; and
B) the determination of the presence, nature and/or the concentration of each ADAR protein contained in said blood sample cells by the implementation of antibodies capable of recognizing specifically said ADAR proteins.

Labelled antibodie can be cited.

Among these antibodies which can be used for this detection or/and quantification, the followed antibodies can be cited but are not limited to:
sc-33179 anti-ADARl (H- 176) polyclonal antibody (Santa-Cruz); or
sc-33180 anti-ADAR2 (H-90) polyclonal antibody (Santa-Cruz).

Western blotting or Elisa method can be used for analysing or quantifying specific protein expression in biological sample. Such methods are well known from the skilled man.

The blots can be detected using antibodies specifically directed against different specific regions or epitope of mouse, human or rat ADAR1 -150 or ADAR1-110, and ADAR2 protein. These antibodies can be polyclonal or monoclonal antibodies and are labelled if necessary. Such antibodies can be developed in laboratory using recombinantADAR protein or fragment thereof as immunogen.

The following examples and also the figures and the legends hereinafter have been chosen to provide those skilled in the art with a complete description in order to be able to implement and use the present invention..

### Legends of the figures

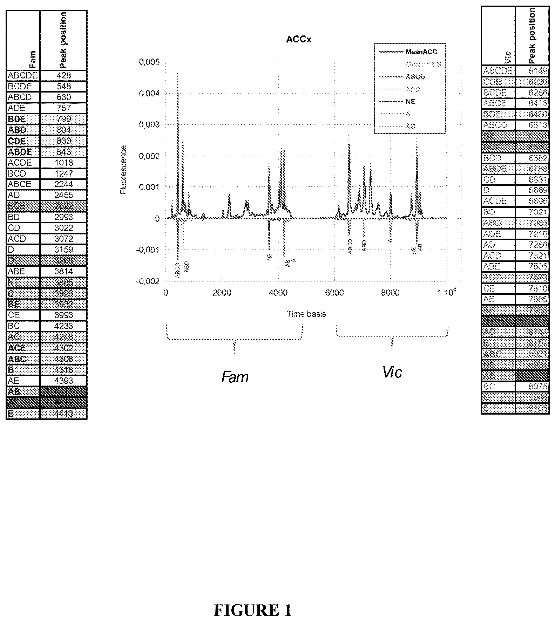

**Figure 1****:** Mean (Black line) of electrophoretic SSCP signals calculated from each individual anterior cingulate cortex signals of a controls group of human subjects (experimental data). The curve corresponding to the mean + SEM of these signals is also presented (green line). The abcissae represents the time basis of 10000 points (6.2 points/second) and the ordinates are given in fluorescence arbitrary units after normalization of the total signal. The presented signal corresponds to the FAM (left part) and VIC (right part) labelled strands. Some typical examples of electrophoresis signals obtained with individual standards are presented as negative in their corresponding FAM and VIC labelling. On the left and right parts of the figure the tables present, applicated to an unique base time, the positions of the different principal electrophoresis peaks (maximum) of each standard single strand of a particular edited isoform labelled with FAM (left) or VIC (right) probes. Two maximum of identified peaks could be separated when their interval was > 15 points of the base time defined above. Note that some non resolved peaks from electrophoretic pattern of one labelled strand are resolved from the other (these cases are identified by the same color).

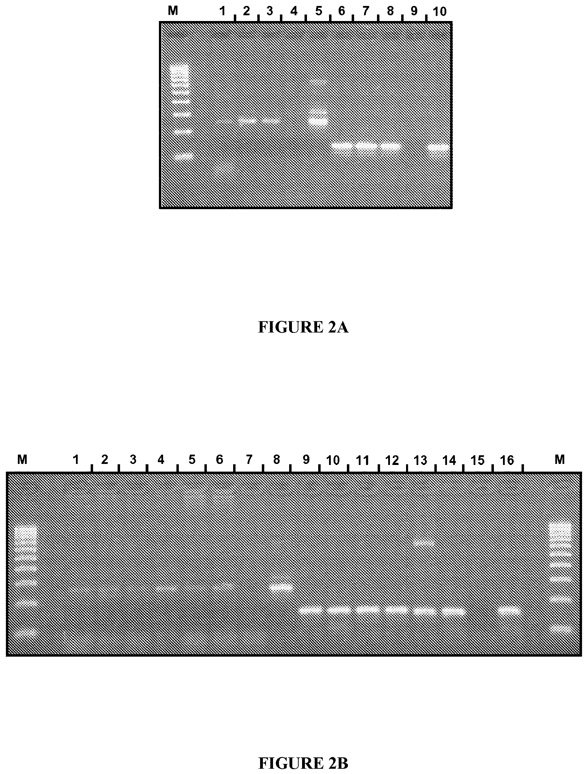

**Figures 2** **A and 2B:** Identification of 5-HT2cR transcripts in Human and mouse skin. (A) cDNAs prepared from Human eyelid polyA+ RNAs (lanes 1-3) were amplified by a first-round PCR using the two specific primers PCR9 and PCR10. A nested-PCR was then conducted on these first products with primers PCR1 8 and PCR2 (lanes 6-8). The resulting products were resolved on a 2% agarose gel. The expected sizes of the amplification products are 250-bp and 127-bp long for first and second PCR, respectively. Negative (lanes 4 and 9) and positive controls (lanes 5 and 10) are shown for each primer set. A 100-bp DNA ladder/marker is indicated by M. (B) cDNAs prepared from Balb/c mouse skin polyA+ RNAs were amplified by a first-round PCR using the two gene specific primers PCR9 and PCR10 (lanes 1-6). A second PCR was then conducted on these first amplifications with primers PCR1 and PCR4 (lanes 9-14). Negative (lanes 7 and 15) and positive controls (lanes 8 and 16) are shown for each primer set. The resulting products were resolved on a 2% agarose gel. The expected sizes of the amplification products are 250-bp and 138-bp long for first and second PCR, respectively. M is for the 100-bp DNA ladder/marker.

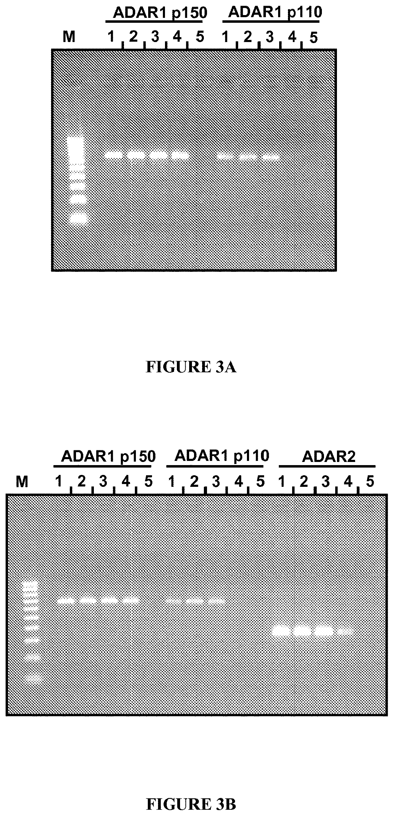

**Figures 3A and 3B****:** The mRNA of the ADARs isoenzymes are clearly identified in Balb/c Mouse Blood and Skin. (A) cDNA prepared from whole-blood RNA (lane 4) and skin RNA (lanes 1-3, corresponding to 3 different times of reverse transcription, i.e 30 min, 2 h, and 4 h respectively) were amplified by PCR with primers specific to the constitutive (pi 10) and inducible forms of ADARl (pl50). The resulting amplification products resolved on a 2% agarose gel are 674-bp (pl50 isoform) and 683-bp (pi 10 isoform) long, respectively. Negative controls (lane 5) and the 100-bp DNA ladder/marker (M) are shown. The boosted contrast allows detection of a faint band corresponding to the constituve form of ADARl transcribed in whole-blood cells (lane 4, pi 10). (B) Same as in (A), but PCR amplifications corresponding to ADAR2 transcripts in skin and whole-blood are presented. The PCR products are 366-bp long. Again, a very faint band corresponding to the constituve form of ADAR1 in whole-blood is observed (lane 4, pi 10).

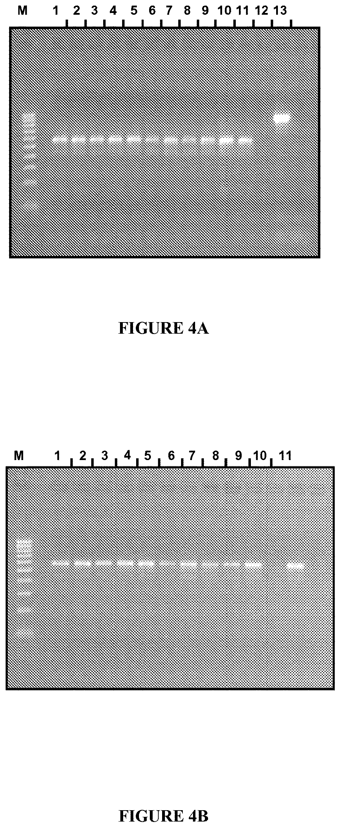

**Figures 4** **A and 4B:** Identification of inducible ADAR 1 transcripts in Human leukocytes. (A) cDNAs prepared from Human peripheral blood leukocytes total RNA (lane 10) and from Human blood fractions normalized libraries (lanes 1-9) were amplified by PCR with primers specific to the inducible form of ADAR1 (pi 50). The resulting products were resolved on a 2% agarose gel. The expected size of the amplification product is 544-bp long. Lanes 1 and 6; 2 and 7; 3 and 8 correspond to resting and activated mononuclear cells, resting and activated CD4+, and resting and activated CD8+ cells, respectively. Lane 4 and 5: resting CD14+ and CD19+ cells. Lane 9: activated CD 19+ cells. Lane 11: cDNA from Human placenta used as a control. The 100-bp DNA ladder/marker is indicated by M. PCR positive (G3PDH primers with Human placental cDNA, lane 13) and PCR negative controls (lane 12) are shown. (B) Duplicate of (A). Lanes 1-9 are equivalent to lanes 1-9 of (A). Lane 10: Human placenta cDNA.

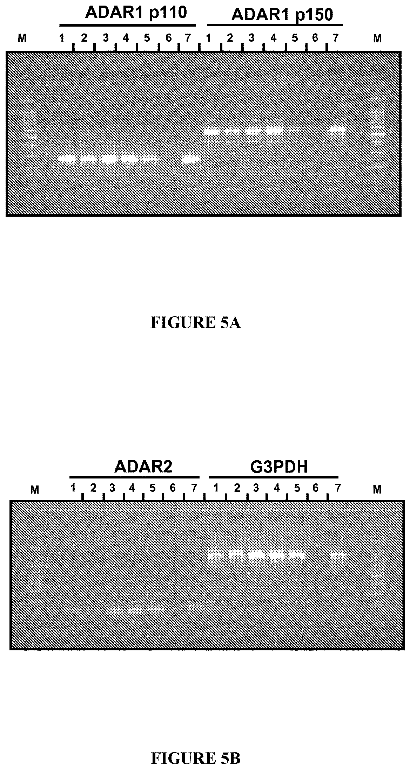

**Figures 5A and 5B****:** Identification of ADAR1 pi 10, ADAR1 pi 50, and ADAR2 transcripts in Human Dorsal Prefrontal Cortex, skin and CD4+ and CD8+ blood cells.
   (A) cDNAs from Human CD4+ and CD8+ blood fractions normalized libraries (lanes 1 and 2); prepared from DPFC total RNA (lanes 3 and 4) and from Human eyelid polyA+ RNA (lane 5) were amplified by PCR using the two specific set of primers EXlB 534p/EX2 804m and EXlA 34p/EX2 578m for ADAR1 pi 10 and ADAR1 pl50 respectively. The resulting products were resolved on a 1.75 % agarose gel. The expected sizes of the amplification products are 270-bp (ADAR1 pi 10) and 566-bp (ADAR1 p 150). Negative (lane 6) and positive controls (lane 7, Human placental cDNA) are shown for each primer set. A 100-bp DNA ladder/marker is indicated by M.
   (B) The same cDNAs as in A) were amplified by PCR using the two gene specific set of primers ADAR2 1274p/ADAR2 1486m and G3PDH-F/G3PDH-R for ADAR2 and
   G3PDH (positive control) respectively. The resulting products were resolved on a 1.75 % agarose gel Negative. The expected size for ADAR2 is 212-bp long.

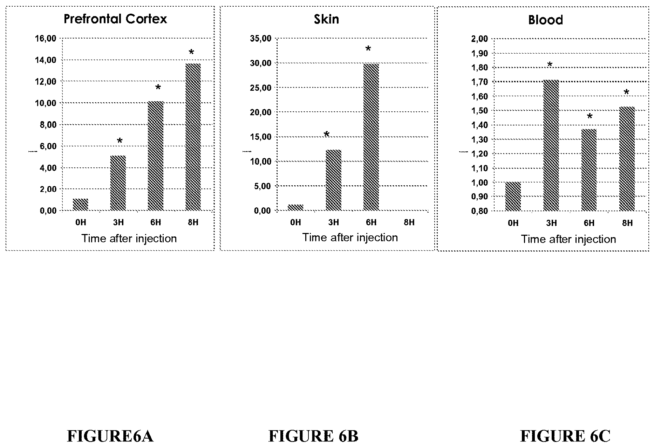

**Figures 6A-6C****:** Evolution of the concentration of ADARl a specific mRNA measured by QPCR in prefrontal cortex (figure 6A), skin (figure 6B) and blood (figure 6C) sample after interferon alpha2a mouse interferon single IP injection at t zero (20000 IU). The effect is expressed as the fold increase from control value normalized at 1. * p<0.05.

### EXAMPLE 1: Strategy implemented

The validation of such a strategy has implicated:
1) The identification of significant alteration of the editing process in human brain in a given pathology and the validation of its pathogenic implication in the pathology by postmortem observations made in convergent pathological models obtained in mouse and/or rat.
2) The identification of peripheral tissues or cell lines easily accessible at a non invasive level which could be used for the diagnostic and therapeutic adjustment.

In the present invention the validation of this strategy was obtained by:
A - The measurement of adequate markers of the editing process to all or part of the implicated editing regulation: 5-HT2cR mRNA editing profiles, markers of the expression of the different iso forms of the editing enzymes: ADARl -150, ADARl-110, ADAR2.

As an example of the results obtained to validate the present invention the table I precises the set of markers which are proposed after their identification in the proposed sources from Human subjects and experimental animals or cell lines:

**Table 1 : Level of expression of 5HT2cR and editing enzymes. We note that in the blood samples the level of expression of the editing enzymes can be easily determined. In skin samples the 5-HT2cR is expressed and is submitted to the editing activity of ADARs 1 and 2 and can complete the investigation of the general state of regulation of the editing of this receptor in the brain samples.**

| | | Brain | Skin | Blood |
|---|---|---|---|---|
| 5-HT2cR | | | | |
| Mouse (Balb/c) | | +++ | + Edited by ADARS1 and 2 | 0 |
| Human | | + | + Edited by ADARS1 and 2 | 0 |
| | | | | |

| ADARs | | | | |
|---|---|---|---|---|
| Mouse | ADAR1-150 | ++ | +++ | ++ |
| | ADAR1-110 | ++ | +++ | + |
| | | | | |
| | ADAR2 | ++ | ++++ | ++ |
| | | | | |
| Human | ADAR1-150 | ++ | ∗∗ | ++ |
| | ADAR1-110 | ++ | ∗∗ | ∗∗ |
| | | | | |
| | | | | |
| | ADAR2 | + | ∗∗ | ^{∗∗} |

For each marker the samples are rapidly extracted with special care for allowing the possibility to execute the determination of the levels of expression of mRNA, the editing profiles and the determination of proteins markers by western blotting from the same tissue or cell sample.

The table 2 summarizes the preferred processes used for the determination of level of expression of the used biomarkers.

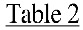

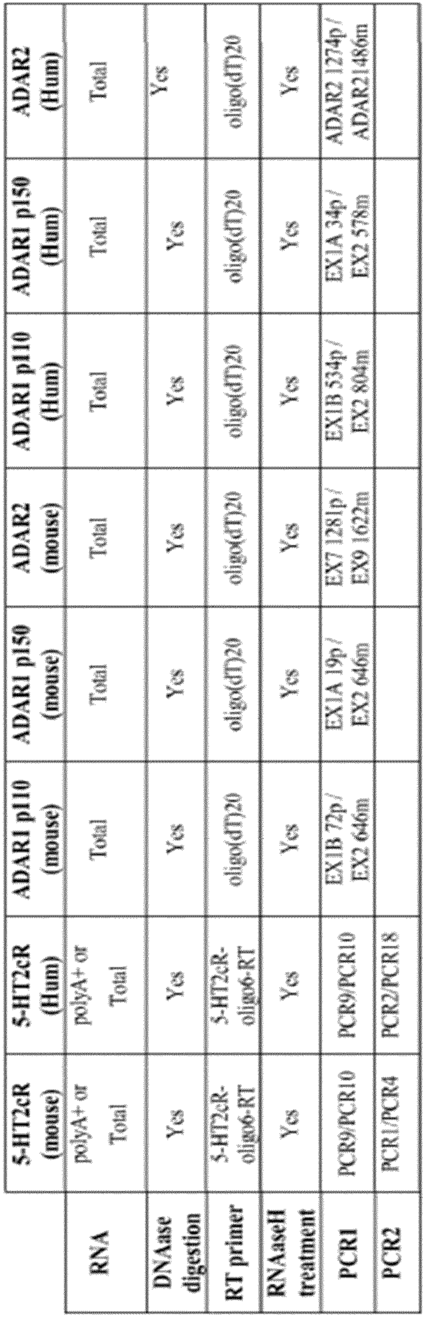

The following examples illustrate typical procedure and results.

### EXAMPLE 2: Obtention of the complete editing profile from one sample of brain tissue (figure 1)

Total RNA was extracted and purified from tissue or cell extracts, according to manufacturer's specifications (Qiagen RNeasy, Mini Kit). The quantity and purity of the extracted RNA were assessed by measuring both the absorbance at 260nm and the 260/280 nm ratio with a GeneQuant spectrophotometer (PharmaciaBiotech). In order to eliminate possible contamination by genomic DNA, 8 µl of each RNA (between 88 ng and 1.3 µg) were then treated with 1 unit of DNase I (Invitrogen) for 15 min at room temperature in a final volume of 10 µl. The reaction was stopped by adding 1 µl of 25mM EDTA and then heated for 10 min at 65°C. The reverse transcription of DNAse I-treated RNAs (10 µl) was performed using 15 units of ThermoScript reverse transcriptase (ThermoScript RT-PCR System, Invitrogen) and Oligo(dT) primers at a final concentration of 2.5 µM.

A first PCR reaction (final volume 25 µl) resulting in a 250 bp fragment, was then carried out on 1 µl of the reverse transcription products with 0.2 unit of Platinum Taq DNA polymerase (ThermoScript RT-PCR system, Invitrogen) and specific primers (forward primer: 5 '-TGTCCCTAGCCATTGCTGATATGC-S ' (SEQ ID NO. 35) and reverse primer: 5 '-GCAATCTTCATGATGGCCTTAGTC-S ' (SEQ ID NO. 36); final concentration of each 0.2µM) located on exon IV and exon V of the Human 5-HT2cR cDNA, respectively. After a denaturing step at 95°C for 3 min, the PCR was brought to its final point after 35 cycles (15s at 95°C; 30s at 600C; 20s at 72°C), and a final elongation step of 2 min at 72°C. Aliquots of the amplification products were used to check the product on a 2% agarose analytic gel.

### Second PCR and separation of single-strand cDNA fragments by Capillary Electrophoresis (CE)

1 µl of a 1/50 dilution of the RT- 1 st PCR products, or the 250 bp cDNA amplified from plasmids harboring the thirty-two standard of human 5-HT2cR (or5HT2CR) isoforms, were used as templates for an additive nested-PCR. These 32 standards, corresponding to the non-edited (NE) and edited isoforms of human 5- HT2cR. Amplifications were performed in a final volume of 20 µl with HPLC-purified fluorescent primers (forward primer: FAM-ATGTGCTATTTTCAACAGCGTCCATC-3' (SEQ ID No. 37); reverse primer: VIC-GC AATCTTCATGATGGCCTT A-3 ' (SEQ ID No. 38); final concentration of each 0.2 µM), and 0.2 unit of Platinum Pfx DNA polymerase (Invitrogen).

The VIC-labelled reverse primer hybridizes to a complementary sequence of the 5-HT2c receptor identical in human, mouse and rat. On the other hand, although used with human samples, the sequence of the FAM-labelled foward primer was designed to be as close as possible to that of the mouse. More precisely, T residues in positions 5 and 6 of the human oligonucleotide sequence (positions 1133 and 1134 of human reference U49516) were changed into G and C, respectively.

Simulations of stochastic folding pathways of both strands of the PCR product obtained with the two primers described above were carried out with the Kinefold server (kinefold.curie.fr). They showed that the lowest free-energy structures obtained for forward and reverse strands - the edited region embedded in the loop of a stem- loop structure, and able to hybridize with a complementary sequence located elsewhere in the whole structure after folding of the stem - were very close to that calculated for a mouse nested-PCR product successfully used for Mouse samples. This set of primers was shown to be optimal for conformational analysis of human 5HTR2C mRNA editing by non denaturing capillary electrophoresis by single strand conformational polymorphism (CE-SSCP).

The amplified fragment is 127 bp-long. As for RT-PCR, after an initial denaturing step of 5 min at 94°C, the amplification reaction was brought to an end with 35 cycles (15s at 94°C; 30s at 55°C; 20s at 68°C) and a final elongation step of 2 min at 68°C. Again, quality of the 127 bp-long amplified fragments were assessed on a 2% agarose gel before subsequent analysis in a 3100 Avant Genetic Analyser (Applied Biosystem).

Fluorescent PCR products corresponding to standard isoforms (1 µl of a 1/100 dilution in DEPC treated water) and samples (1 µl of a 1/30 dilution) diluted in 11 µl of deionized formamide were added to a mixture of ROX labelled migration standards (MWG-BIOTECH, AG) (0.5 µl each) covering the whole range of the electrophoregram retention times. These ROX standards were used for CE calibration and subsequently to obtain correct superimposition of standards and samples peaks. After denaturing for 2 min at 95°C, samples were immediately chilled on ice. Non-denaturing CE was carried out in an ABI PRISM 3100-Avant Genetic Analyser (Applied Biosystems) through 80 cm-long capillaries filled with 7% "POP Conformational Analysis Polymer" (Applied Biosystems), IX TBE and without glycerol. After a pre-run performed at 15 kV for 3 min, samples were injected for 15s at 2 kV, and electrophoresis was run for 105 min at 15 kV at a controlled temperature of 200C. Under these conditions, each of the thirty-two possible iso forms were clearly resolved as a result of the single ssDNA conformation obtained with either the FAM-labelled or the VIC-labelled strand. The different retention times were used for unambiguous identification of the iso forms.

### Identification and relative quantification of each isoform in each brain sample

The Electrophoretic Signal was then processed using an in-house software. First, the time basis of electrophoretic profiles of each sample was adjusted using the ROXlabelled strands of the migration standards. This allowed FAM- and VIC-labeled strands to precisely deconvolute the standards and samples signals in a unique time basis. Background was then adjusted and subtracted and then total area under each signal normalized.

The relative proportion of each isoform was processed by a best fitting of each deconvoluted and normalized analytical signal of the brain samples. It was performed by the iterative adjustment of the integrated signal represented by the 32 similarly deconvoluted and normalized standard analytical signals. The calculation was based on

The hypothesis that the SSCP signal S(t) = ΣgᵢRᵢ(t) in which Rᵢ(t), with i ∈ {1,.., N}, are the standard signals and gᵢ the % of each of them in the signal. The best fit minimized the sum of squares due to error (SSE) SSE= ∫[S(t)-ΣgᵢRᵢ(t) ] ² and was controlled by the least square statistical analysis.

The result of this best fitting was statistically evaluated after calculation of the γ2 value such as r ²=1 - SSE/SSM in which SSM is the Sum of Square about Mean such as SSM =Σ (S(t) - S)²
(S(t) - S) . The maximum theoretical best fit would give an r ²= 1.

All experiments were carried out under blind conditions and all samples were assayed in the same batch for RT-PCR and second PCR reactions. The best fitting results yielded a specific editing profile for each individual sample, which was determined by the percentage of each edited and non edited form of the total analytical signal. These initial values were used for statistical analyses.

This method gives the proportion of each expressed mRNA isoform expressed as the percentage of the total of 5-HT2c receptor present in the extract.

As an example is given here the table of identification of the 32 human iso forms in which each FAM and VIC labelled strands gives a set of retention time (see figure 1). It is easy to note that the use of the two strands can solve the total identification of the 32 standards iso forms.

The main advantage of this processing is to give a complete quantitative estimation of the distribution of the expressed of SHT2cR iso forms (editing profile) in a given concentration of SHT2cR mRNA. This is obtained from one single assay and allows to easily determine the characteristics of this profile in a given situation.

With this technique it has been possible to demonstrate in a group of 6 depressed patients having committed suicide a specific signature which characterized the depressed group of patients. This signature gives interesting information about the dysregulation of editing process occurring in the dorsal prefrontal regions of the brain and strongly support the interest to explore the steady state of editing enzymes in skin and blood samples of depressed patients.

### EXAMPLE 3: SHT2CR expression in Human and Mouse skin

Skin, because the dermal presence of 5-HT2c receptors and editing enzymes could be an interesting source for measuring at the periphery both the 5-HT2cR editing and the level of expression of the editing enzymes.

Figure 2 represents a typical control of RTPCRs performed after extraction of polyA+ RNAs from Human (A) or Mouse (B) skin samples.

The application of the capillary electrophoresis separation of the SSCP products of the second nested PCR products led to achieve the demonstration that, in the human and Mouse skin, the ADAR1 and ADAR2 editing enzymes were active and that pathological or physiopathological states could modify in this peripheral tissue the editing regulation of the 5-HT2cR.

### EXAMPLE 4: Location and nature of the expression of ADARl and ADAR 2 iso forms As predicted by the preceding validation experiments, the expression of ADAR1 and ADAR 2 iso forms were found expressed in the skin samples of Mouse and Human Skin.

The figure 3 shows an example of control of the RT/PCR identification of ADARl-150, ADAR1-110 and ADAR 2 in experiments performed after extraction of Mouse total blood or skin RNAs.

In Man it was also possible to identify and to easily quantify the level of expression of editing enzymes after collection of a small volume of blood.

With a typical yield of 1-2 × 10⁶ leukocytes per ml of freshly collected blood, a volume of 5 ml allows to isolate enough total RNA for reverse transcription reactions. Blood samples (5ml) are collected into heparinized tubes. The following steps of the protocol must be immediately carried out under sterile conditions. Dilute the anticoagulated sample material with an equal volume of 0.9% NaCl sterile solution, or IX PBS sterile solution, or RPMI 1640 sterile culture medium. Separation medium (eg Ficoll-Paque Plus, GE Healthcare Bio-Sciences AB, ref : 17-1440-02 or 17-1440-03) must be warmed-up to room temperature just before use and protected from light. Fill 15 ml LeucoSep tube (Greiner Bio-One, ref : 163 289 or 163 290) with 3 ml of separation medium. Centrifugate for 30 s at 1000 g and room temperature (the separation medium is then below the porous barrier of the tube). When using LeucoSep tubes that are pre-filled with separation medium, the aforementioned steps can be cancelled (ref: 163 288 or 227 288). Simply warm-up the tubes to RT. Pour carefully the anticoagulated, diluted material sample (1 :2 in balanced salt solution or RPMI 1640, see above) into the 15 ml LeucoSep tube. Centrifugate 10 minutes at 1000 g and room temperature, or 15 minutes at 800 g and room temperature in a swinging bucket rotor. Switch-off brakes of the centrifuge

After centrifugation, harvest the enriched cell fraction (lymphocytes/PBMCs = white ring) by means of a Pasteur pipette. Wash the enriched cell fraction with 10 ml of IX PBS sterile solution, subsequently centrifugate 10 minutes at 250 g. Repeat washing step twice. For the last centrifugation, pellets must be collected into microcentrifuge tubes (1.5 ml Eppendorf tubes) via resuspension in 1 ml IX PBS sterile solution. After the last centrifugation (10 minutes at 250 g and room temperature) discard the supernatant and quickly cover the "dryed" pellets of enriched cell fractions with RNAlater RNA stabilization Reagent (Qiagen, ref : 76104 or 76106). If the submerged pellets are stored at 2-8°C, the lymphocytes/PBMCs gene expresion profile can be stabilized up to 4 weeks at this temperature. If transporting samples in RNAlater reagent, ensure that the pellets always remain submerged in the reagent. Either keep the tubes upright during transport or fill the tubes completely with the stabilization solution. During transport tubes can be kept in a polystyrene box filled with blue ice packs. A better solution could be to directly lyse the pellets of the enriched cell fraction in 1 ml of TRIzol Reagent (Invitrogen, ref : 15596-026), kept and sent at room temperature. As this lysis reagent contains phenol, samples tubes must be tightly closed. Actually, as described by the furnisher (Invitrogen), the leukocytes lysate in TRIzol reagent (see above) allows later extractions of both total RNA (aqueous phase) and proteins (organic phase). Tubes could be sent at room temperature or in dry ice.

An example of validation is given on figure 4 in which is presented the control of the RTPCR products of editing enzymes in human brain, skin and CD4 and CD8 blood cells, samples.

It is thus possible, in Human, to correctly analyse the expression of the editing enzymes in Brain (post mortem studies), Skin and Blood samples (diagnostic studies) as illustrated on the control experiment presented on figure 5.

All the elements of the table 2 being verified, it becomes possible to elucidate the precise conditions and the limits of the analysis of blood and skin editing enzyme expression and 5-HT2/C editing profile in skin as bio markers in diagnostic and treatment of several pathological states in human with a deep validation coming from adequate physio- or pharmaco-pathological models.

### EXAMPLE 5: Analysis of the iso forms of the 5-HT2c receptor in post mortem suicide-depressed patients brain samples compared with patient controls samples

The analytical process illustrated in figure 1 allows analysis of all iso forms of the 5-HT2c receptor in post mortem brain samples. A study of 6 patient controls and carefully-selected suicide-depressed patients showed that: (1) specific brain regions show a specific pattern of distribution of the edited and non-edited 5-HT2CR iso forms, and (2) this distribution pattern is significantly altered in the human anterior cingulate cortex and dorsal prefrontal cortex, which are involved in the pathophysiology of major depression. These changes in the isoform signature are illustrated in table 3, which shows the effects observed in the anterior cingulate cortex. Importantly, this technique can measure dynamic changes in isoform prevalence in both directions-increases or decreases compared to controls, providing insight into the potential enzyme dysfunction underlying the changes. Here, for example, ADAR I activity (which specifically edits the A and B sites and, with less specificity, the C site) is up-regulated.

Table 3: Editing profiles obtained in controls and suicide depressed patients. The total editing profile was measured in patients from both groups (n=6 in each). Results represent the isoform prevalence as a percent of all iso forms and given as the mean ± SEM. The two aspects of the signature-the distribution of all iso forms and the distribution according to individual editing sites- were analyzed by ANOVA II.

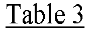

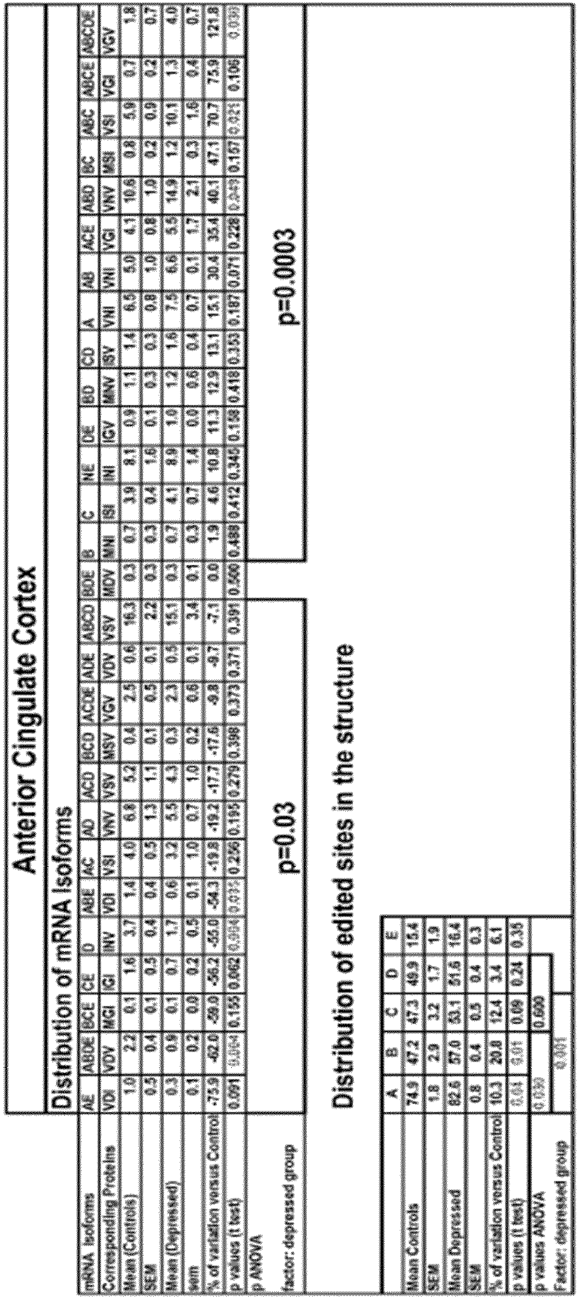

### EXAMPLE 6: Interferon alpha2 treatment in mouse induces significant increase in ADAR1 expression in blood. This peripheral effect is also identified in the skin and in brain.

This experiment was performed in Balb/cJ Mouse. A dose of 20,000 IU of mouse alpha interferon was injected by IP route and mice groups (n=8) killed at time 3, 6 and 8 hours after injection. A control group (n=8) was killed at time zero. The blood, skin and brain were rapidly processed to avoid RNA degradation. Total RNA was extracted from each tissue sample and specific mRNA coding for inducible ADARl was quantified by QPCR using GAPDH endogenous gene as reference. The results are summarized on figure 6. They clearly show that when a significant increase in ADAR 1 expression is observed in blood samples of interferon treated mice, an amplified response is also observed in skin samples and in prefrontal cortex.

Additionally, the editing profile of the 5-HT2cR was determined according to the methods previously described (see example 2) in the prefrontal area of control and interferon treated mice killed at 8 hours. In table 4 it is easy to see that:
1) Following the induction of the ADAR 1 expression occurring rapidly in brain, in these experimental conditions, a significant early alteration of the editing process of the 5-HT2cR is seen at 8 Hours. A group of 12 edited iso forms including the ABCD and ACD iso forms was found significantly increased. They represent more than 30 % of the total 5-HT2cR mRNA.
2) The analysis of the proportions of edited sites in this group clearly exhibit a significant increase in the proportions of A, B, and C sites found edited. They can be interpreted as mainly resulting from a greater activity of ADARl . This is confirmed when the analysis is restricted to the isoforms of this group which are exclusively due to the activity of ADAR 1.

Table 4: Analysis of the alteration of the editing profile of 5-HT2cR mRNA after interferon treatment. In each individual, the total profile of editing was measured as the proportion of each edited isoforms in the total specific mRNA of the receptor. Presented results are the mean ± SEM of controls and interferon treated mice killed at 8 Hours after injection. P values were calculated from Student test. Are thus presented : the sum of the edited iso forms found increased in the interferon treated goup, the proportion of the A,B,C,D and E sites found edited in this group of isoforms and the relative proportion of mRNA represented by the isoforms of this group which is exclusively the result of a specific action of ADARl.

Finally it becomes reasonable to propose that the measure of changes in ADARs expression at the periphery (blood) could predict important alteration of editing in the brain which could explain the secondary effects on mood of several already used treatments in several therapeutic fields.

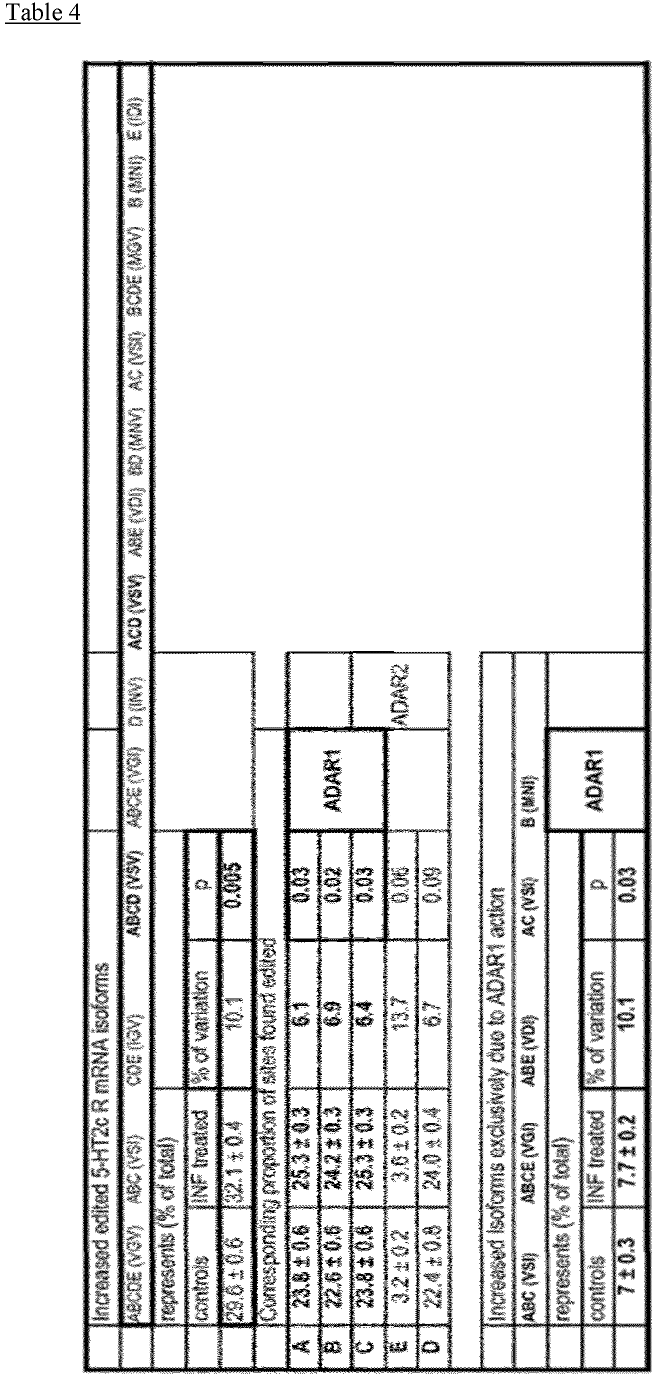

## Claims

1. A method implementing a single biological sample selected from the group of biological sample consisting of peripherical tissues containing cells for evaluating the pathological alteration of a mRNA editing in the brain and wherein said mRNA editing is an ADAR dependent A to I mRNA editing;
- wherein said peripherical tissue containing cells is selected from the group consisting whole blood sample or blood sample containing buffy coat;
- wherein said edited mRNA is 5HTR2C mRNA;
- evaluating the pathological alteration of the 5HTR2C mRNA editing in the brain by determining the ADAR expression products ADAR1, isoforms 150 and/or 110, and the ADAR2 gene expression products expressed in said sample.

2. Method for identifying in vitro whether a patient presents a pathology or is at risk to develop a pathology related to an alteration of the mechanism of the mRNA editing of the 5HTR2C, wherein this method comprising the following steps of:
a) determining the ADAR expression products ADAR1, isoforms 150 and/or 110, and the ADAR2 gene expression products in a biological sample containing blood cells obtained from the patient to be tested;
b) identifying whether said patient presents or is at risk to develop such a pathology by comparing the quantity of the ADARs expressed in said sample with expressed ADARs profil obtained for normal patients or for patients exhibiting pathologies related to an alteration of the mechanism of this mRNA editing.

3. The method of claims 1 or 2, wherein said pathology is selected from the group consisting of mental disorders, schizophrenia, depression, depressed suicide or abnormal feeding behaviour.

4. The method of claims 1 or 2, wherein said pathology is selected from the group consisting of depression, and depressed suicide behaviour.

5. The method of claim1 for determining in vitro whether a pathology exhibited by a patient is related to an alteration of the mechanism of the mRNA editing of the 5HTR2C, wherein this method comprising the following steps of:
a) determining the quantity of the ADARs expressed in a biological sample containing blood cells obtained from the patient;
b) identifying whether said patient presents or is at risk to develop such a pathology by comparing the quantity of the ADARs expressed in said sample with expressed ADARs profil obtained from normal patients or from patients exhibiting pathologies known to be not related to an alteration of the mechanism of this mRNA editing.

6. A method for identifying in vitro an agent that modulates in vivo the editing of the 5HTR2C mRNA in a mammal, comprising the following step of:
- comparing the ADAR expression products ADAR1, isoforms 150 and/or 110, and the ADAR2 gene expression products in a biological sample containing blood cells obtained from the mammal after treatment with said agent with the ADAR expression products obtained from control blood sample obtained from said mammal.

7. A method for identifying in vitro an agent that modulates the editing of the 5HTR2C mRNA in a mammal, comprising the following steps of:
a) contacting a biological sample containing mammalian blood cells line in the presence of a candidate modulator of said 5HTR2C mRNA editing; and
b) determining the effects of said modulator on the ADAR expression products ADAR1, isoforms 150 and/or 110, and the ADAR2 gene expression products expressed in said sample of blood cells, by comparing the quantity of the ADARs expressed obtained from the biological sample in step a) with the quantity of the ADARs expressed obtained from control tissue sample of bloods cells of said mammal.

8. A method for determining in vitro in a patient the efficiency of a drug used for the prevention or for the treatment of a pathology related to an alteration of the mechanism of the mRNA editing of the 5HTR2C, comprising the following steps of:
a) determining the ADAR expression products ADAR1, isoforms 150 and/or 110, and the ADAR2 gene expression products in a blood sample containing blood cells obtained from the patient; and
b) determining the efficiency of said drug by comparing the ADAR expression products ADAR1, isoforms 150 and/or 110, and the ADAR2 gene expression products obtained in step a) with the ADAR expression products obtained from the patient during or/and after the treatment with said drug,
wherein a modulation of the ADAR expression products expressed resulting to ADAR expression products expressed close or equal to this observed for normal patients being significant of the efficiency of the treatment.

9. Method for determining if a patient responds or does not respond to a treatment of a pathology resulting or provoking by the alteration of the mechanism of the mRNA editing of the 5HTR2C, comprising the following steps of:
a) determining the ADAR expression products ADAR1, isoforms 150 and/or 110, and the ADAR2 gene expression products in a blood sample containing blood cells obtained from the patient before the beginning of the treatment;
b) determining if the patient responds or not responds to the treatment by observing the modification of the ADAR expression products after a period of treatment by comparing with the quantity of the ADARs expression products obtained in step a) before the beginning of the treatment.

10. The method according to claims 1 to 9 wherein the patient or the mammal is human, a mouse or a rat.

11. A method according to claims 1 to 10, wherein the ADAR expression products are ADAR1 isoforms 150 and/or 110, and the ADAR2 gene expression products of the human gene encoding the ADAR1, isoforms 150-kD and/or 110-kD protein, and the ADAR2 protein.

12. A method according to claims 1 to 11, wherein the ADAR expression products are the ADAR mRNAs.

13. A method according to claim 12, wherein), the determination of the ADAR mRNA is carried out by a method which comprises the following steps:
A) extraction of the total RNAs of said blood sample cells, followed, where appropriate, by purification of the mRNAs;
B) reverse transcription of the RNAs extracted in step A); and
C) PCR amplification of the cDNAs obtained in step B) using at least a pair of primers specific for each of the ADAR mRNA to be quantified and/or qualitatively analysed.

14. A method according to claim 13, wherein in step C), the pair of primers specific for the ADAR mRNA PCR amplification are selected from the group consisting of:
- for human ADAR1 -150 isoform mRNA amplification:
Forward: 5 '-GCCTCGCGGGCGCAATGAATCC-S ' (SEQ ID NO. 41),
Reverse: 5 '-CTTGCCCTTCTTTGCCAGGGAG-S ' (SEQ ID NO. 42);
- for human ADAR1-110 isoform mRNA amplification:
Forward: 5 '-CGAGCCATCATGGAGATGCCCTCC-3 ' (SEQ ID No. 43),
Reverse: 5 '-CATAGCTGCATCCTGCTTGGCCAC-S ' (SEQ ID NO. 44);
- for human ADAR2 mRNA amplification:
Forward: 5 '-GCTGCGCAGTCTGCCCTGGCCGC-S ' (SEQ ID NO. 45),
Reverse: 5 '-GTCATGACGACTCCAGCCAGCAC-S ' (SEQ ID NO. 46);
- for mouse ADAR1 -150 isoform mRNA amplification:
Forward: 5 '-GTCTCAAGGGTTCAGGGGACCC-S ' (SEQ ID NO. 47),
Reverse: 5 '-CTCCTCTAGGGAATTCCTGGATAC-S ' (SEQ ID NO. 48);
- for mouse ADAR1-110 isoform mRNA amplification:
Forward: 5 '-TCACGAGTGGGCAGCGTCCGAGG-S ' (SEQ ID NO. 49),
Reverse: 5 '-CTCCTCTAGGGAATTCCTGGATAC-3 ' (SEQ ID No. 48); and
- for mouse ADAR2 mRNA amplification:
Forward: 5 '-GCTGCACAGTCTGCCTTGGCTAC-S ' (SEQ ID NO. 50),
Reverse: 5 '-GCATAAAGAAACCTGAGCAGGGAC-S ' (SEQ ID NO. 51).

15. A method according to claims 1 to 14, wherein the ADAR expression products are the ADAR proteins.

16. A method according to claim 15, wherein the determination of the ADAR proteins is carried out by a method which comprises the following steps:
A) the extraction of the total proteins contained in said blood sample cells, followed, where appropriate, by a step of proteins purification; and
B) the determination of the presence and/or the concentration of each ADAR protein contained in said blood sample cells by the implementation of antibodies capable of recognizing specifically said ADAR proteins.

## Patentansprüche

1. Verfahren zum Implementieren einer einzelnen biologischen Probe, aus der Gruppe biologischer Proben ausgewählt, bestehend aus peripheren Geweben, die Zellen enthalten, zum Bewerten der pathologischen Veränderung eines mRNA-Editing in dem Gehirn, und wobei das mRNA-Editing eine ADAR-abhängige A bis I mRNA-Editing ist;
- wobei das periphere Gewebe, Zellen enthält, aus der Gruppe ausgewählt, bestehend aus einer Vollblutprobe oder einer Blutprobe, die Leukozytenfilm enthält;
- wobei die bearbeitete mRNA 5HTR2C mRNA ist;
- Bewerten der pathologischen Veränderung des 5HTR2C mRNA-Editings in dem Gehirn durch Bestimmen der ADAR-Expressionsprodukte ADAR1, Isoformen 150 und/oder 110 und der ADAR2-Genexpressionsprodukte, die in der Probe exprimiert werden.

2. Verfahren zur Identifizierung in-vitro, ob ein Patient eine Pathologie aufzeigt oder gefährdet ist, eine Pathologie zu entwickeln, die mit einer Veränderung des Mechanismus des mRNA-Editings des 5HTR2C zusammenhängt, wobei dieses Verfahren die folgenden Schritte umfasst:
a) Bestimmen der ADAR-Expressionsprodukte ADAR1, Isoformen 150 und/oder 110 und der ADAR2-Genexpressionsprodukte in einer biologischen Probe, die Blutzellen enthält, die von dem zu testenden Patienten erhalten wurden;
b) Identifizieren, ob der Patient eine solche Pathologie aufzeigt oder gefährdet ist, eine solche Pathologie zu entwickeln, durch Vergleichen der Menge der ADARs, die in der Probe mit exprimierten ADAR-Profilen exprimiert werden, die für normale Patienten oder für Patienten, die Pathologien vorweisen, die mit einer Veränderung des Mechanismus dieses mRNA-Editings zusammenhängen, erhalten werden.

3. Verfahren nach Ansprüchen 1 oder 2, wobei die Pathologie aus der Gruppe ausgewählt ist, bestehend aus psychischen Störungen, Schizophrenie, Depression, depressivem Suizid, oder abnormalem Essverhalten.

4. Verfahren nach Ansprüchen 1 oder 2, wobei die Pathologie aus der Gruppe ausgewählt ist, bestehend aus Depressionen und depressivem Suizidverhalten.

5. Verfahren nach Anspruch 1 zur Bestimmung in-vitro, ob eine Pathologie, die von einem Patienten exprimiert werden, mit einer Veränderung des Mechanismus des mRNA-Editings des 5HTR2C zusammenhängt, wobei dieses Verfahren die folgenden Schritte umfasst:
a) Bestimmen der Menge der ADARs, die in einer biologischen Probe exprimiert werden, die Blutzellen enthält, die von dem Patienten erhalten wurden;
b) Identifizieren, ob der Patient eine solche Pathologie aufzeigt oder gefährdet ist, eine solche Pathologie zu entwickeln, durch Vergleichen der Menge der ADARs die in der Probe exprimiert werden mit exprimierten ADAR-Profilen, die von normalen Patienten oder von Patienten erhalten werden, die Pathologien vorweisen, die dafür bekannt sind, nicht mit einer Veränderung des Mechanismus dieser mRNA-Editings zusammenzuhängen.

6. Verfahren zum Identifizieren eines Mittels in-vitro, das das Editing der 5HTR2C mRNA in einem Säugetier in vivo moduliert, umfassend den folgenden Schritt:
- Vergleichen der ADAR-Expressionsprodukte ADAR1, Isoformen 150 und/oder 110 und der ADAR2-Genexpressionsprodukte in einer biologischen Probe, die Blutzellen enthält, die von dem Säugetier nach Behandlung mit dem Mittel erhalten wurden, mit den ADAR-Expressionsprodukten, die aus einer von dem Säugetier erhaltenen Kontrollblutprobe erhalten wurden.

7. Verfahren zum Identifizieren eines Mittels in-vitro, das das Editing der 5HTR2C mRNA in einem Säugetier in vivo moduliert, umfassend die folgenden Schritte:
a) Inberührungbringen einer biologischen Probe, die Blutzellen von Säugetieren enthält, in Gegenwart eines Kandidaten-Modulators des 5HTR2C mRNA-Editings; und
b) Bestimmen der Wirkungen des Modulators auf die ADAR-Expressionsprodukte ADAR1, Isoformen 150 und/oder 110 und die ADAR2-Genexpressionsprodukte, die in der Probe von Blutzellen exprimiert werden, durch Vergleichen der Menge der exprimierten ADARs, die von der biologischen Probe in Schritt a) mit der Menge der exprimierten ADARs, aus einer Kontrollgewebeprobe von Blutzellen des Säugetiers erhalten wurden.

8. Verfahren zum Bestimmen der Wirksamkeit eines Arzneimittels in-vitro bei einem Patienten, das für die Vorbeugung oder die Behandlung einer Pathologie verwendet wird, die mit einer Veränderung des Mechanismus des mRNA-Editings des 5HTR2C zusammenhängt, umfassend die folgenden Schritte:
a) Bestimmen der ADAR-Expressionsprodukte ADAR1, Isoformen 150 und/oder 110 und der ADAR2-Genexpressionsprodukte in einer Blutprobe, die Blutzellen enthält, die von dem Patienten erhalten wurden; und
b) Bestimmen der Wirksamkeit des Arzneimittels durch Vergleichen der ADAR-Expressionsprodukte ADAR1, Isoformen 150 und/oder 110 und der in Schritt a) erhaltenen ADAR2-Genexpressionsprodukte mit den ADAR-Expressionsprodukten, die von dem Patienten während oder/und nach der Behandlung mit diesem Arzneimittel erhalten wurden,
wobei eine Modulation der ADAR-Expressionsprodukte exprimiert wird, die zu ADAR-Expressionsprodukten führt, die nahe oder gleich zu dieser exprimiert werden, die für normale Patienten zu beobachten sind, die signifikant für die Wirksamkeit der Behandlung ist.

9. Verfahren zum Bestimmen, ob ein Patient auf eine Behandlung einer Pathologie anspricht oder nicht anspricht, die durch die Veränderung des Mechanismus des mRNA-Editings des 5HTR2C führt oder hervorgerufen wird, umfassend die folgenden Schritte:
a) Bestimmen der ADAR-Expressionsprodukte ADAR1, Isoformen 150 und/oder 110 und der ADAR2-Genexpressionsprodukte in einer Blutprobe, die Blutzellen enthält, die von dem Patienten vor Beginn der Behandlung erhalten wurden;
b) Bestimmen, ob der Patient auf die Behandlung durch Beobachten der Modifikation der ADAR-Expressionsprodukte nach einer Behandlungsdauer durch Vergleichen mit der Menge der ADARs-Expressionsprodukte, die in Schritt a) erhalten wurden, vor Beginn der Behandlung anspricht oder nicht anspricht.

10. Verfahren nach den Ansprüchen 1 bis 9, wobei der Patient oder das Säugetier ein Mensch, eine Maus oder eine Ratte ist.

11. Verfahren nach den Ansprüchen 1 bis 10, wobei die ADAR-Expressionsprodukte ADAR1-Isoformen 150 und/oder 110 und die ADAR2-Genexpressionsprodukte des menschlichen Gens, das das ADAR1-Isoformen 150-kD und/oder 110-kD-Protein und das ADAR2-Protein codiert.

12. Verfahren nach den Ansprüchen 1 bis 11, wobei die ADAR-Expressionsprodukte die ADAR-mRNAs sind.

13. Verfahren nach Anspruch 12, wobei), die Bestimmung der ADAR-mRNA durch ein Verfahren durchgeführt wird, das die folgenden Schritte umfasst:
A) Extraktion der gesamten RNAs der Blutprobenzellen, gegebenenfalls gefolgt von einer Reinigung der mRNAs;
B) reverse Transkription der in Schritt A) extrahierten RNAs; und
C) PCR-Amplifikation der in Schritt B) erhaltenen cDNAs unter Verwendung von mindestens einem Paar von Primern, das für jede der ADAR mRNA quantifiziert und/oder qualitativ analysiert werden soll.

14. Verfahren nach Anspruch 13, wobei in Schritt C), das Paar von Primern für die ADAR-mRNA-PCR-Amplifikation aus der Gruppe spezifisch ausgewählt ist, bestehend aus:
- für menschliche ADAR1-150-lsoform mRNA-Amplifikation:
Forward: 5 '-GCCTCGCGGGCGCAATGAATCC-S ' (SEQ ID NO. 41),
Reverse: 5 '-CTTGCCCTTCTTTGCCAGGGAG-S ' (SEQ ID NO. 42);
- für die menschliche ADAR1-110 isoform mRNA-Amplifikation:
Forward: 5 '-CGAGCCATCATGGAGATGCCCTCC-3 ' (SEQ ID NO. 43),
Reverse: 5 '-CATAGCTGCATCCTGCTTGGCCAC-S ' (SEQ ID NO. 44);
- für die menschliche ADAR2 mRNA-Amplifikation:
Forward: 5 '-GCTGCGCAGTCTGCCCTGGCCGC-S ' (SEQ ID NO. 45),
Reverse: 5 '-GTCATGACGACTCCAGCCAGCAC-S' (SEQ ID NO. 46);
- für Maus ADAR1-150-Isoform mRNA-Amplifikation:
Forward: 5 '-GTCTCAAGGGTTCAGGGGACCC-S ' (SEQ ID NO. 47),
Reverse: 5 '-CTCCTCTAGGGAATTCCTGGATAC-S ' (SEQ ID NO. 48);
- für Maus ADAR1-110-lsoform mRNA-Amplifikation:
Forward: 5 '-TCACGAGTGGGCAGCGTCCGAGG-S ' (SEQ ID NO. 49),
Reverse: 5 '-CTCCTCTAGGGAATTCCTGGATAC-3 ' (SEQ ID No. 48); und
- für Maus-ADAR2 mRNA-Amplifikation:
Forward: 5 '-GCTGCACAGTCTGCCTTGGCTAC-S ' (SEQ ID NO. 50),
Reverse: 5 '-GCATAAAGAAACCTGAGCAGGGAC-S ' (SEQ ID NO. 51).

15. Verfahren nach den Ansprüchen 1 bis 14, wobei die ADAR-Expressionsprodukte die ADAR-Proteine sind.

16. Verfahren nach Anspruch 15, wobei die Bestimmung der ADAR-Proteine durch ein Verfahren durchgeführt wird, das die folgenden Schritte umfasst:
A) die Extraktion der gesamten Proteine, die in den Blutprobenzellen enthalten sind, gegebenenfalls, von einem Schritt der Proteinreinigung gefolgt; und
B) die Bestimmung der Gegenwart und/oder der Konzentration jedes ADAR-Proteins, das in den Blutprobenzellen enthalten ist, durch die Implementierung von Antikörpern, die in der Lage sind, die ADAR-Proteine spezifisch zu erkennen.

## Revendications

1. Procédé mettant en oeuvre un échantillon biologique unique sélectionné dans le groupe d'échantillons biologiques constitué de tissus périphériques contenant des cellules pour évaluer l'altération pathologique d'une édition d'ARNm dans le cerveau et dans lequel ladite édition d'ARNm est une édition d'ARNm A à I dépendant ADAR ;
- dans lequel ledit tissu périphérique contenant des cellules est choisi dans le groupe constitué d'un échantillon de sang entier ou d'un échantillon de sang contenant une couche leuco-plaquettaire ;
- dans lequel ledit ARNm édité est l'ARNm 5HTR2C ;
- l'évaluation de l'altération pathologique de l'édition d'ARNm 5HTR2C dans le cerveau en déterminant les produits d'expression ADAR ADAR1, isoformes 150 et/ou 110, et les produits d'expression génique ADAR2 exprimés dans ledit échantillon.

2. Procédé destiné à l'identification in vitro du fait de savoir si un patient présente une pathologie ou est à risque de développer une pathologie liée à une altération du mécanisme d'édition d'ARNm du 5HTR2C, dans lequel ce procédé comprenant les étapes suivantes :
a) de détermination des produits d'expression ADAR ADAR1, isoformes 150 et/ou 110, et les produits d'expression génique ADAR2 dans un échantillon biologique contenant des cellules sanguines obtenues à partir du patient à tester ;
b) d'identification du fait de savoir si ledit patient présente ou est à risque de développer une telle pathologie en comparant la quantité des ADAR exprimées dans ledit échantillon avec le profil d'ADAR exprimée obtenu pour des patients normaux ou pour des patients présentant des pathologies liées à une altération du mécanisme de cette édition d'ARNm.

3. Procédé selon les revendications 1 ou 2, dans lequel ladite pathologie est choisie dans le groupe constitué de troubles mentaux, de schizophrénie, de dépression, de suicide dépressif ou de comportement alimentaire anormal.

4. Procédé selon les revendications 1 ou 2, dans lequel ladite pathologie est choisie dans le groupe constitué de la dépression et du comportement suicidaire dépressif.

5. Procédé selon la revendication 1 destiné à la détermination in vitro du fait de savoir si une pathologie présentée par un patient est liée à une altération du mécanisme de l'édition d'ARNm du 5HTR2C, dans lequel ce procédé comprenant les étapes suivantes :
a) de détermination de la quantité des ADAR exprimées dans un échantillon biologique contenant des cellules sanguines obtenues à partir du patient ;
b) d'identification du fait de savoir si ledit patient présente ou est à risque de développer une telle pathologie en comparant la quantité des ADAR exprimées dans ledit échantillon avec le profil d'ADAR exprimée obtenu à partir de patients normaux ou de patients présentant des pathologies connues pour ne pas être liées à une altération du mécanisme de cette édition d'ARNm.

6. Procédé d'identification in vitro d'un agent qui module in vivo l'édition d'ARNm 5HTR2C chez un mammifère, comprenant l'étape suivante :
- de comparaison des produits d'expression ADAR ADAR1, isoformes 150 et/ou 110, et les produits d'expression génique ADAR2 dans un échantillon biologique contenant des cellules sanguines obtenues à partir du mammifère après traitement avec ledit agent avec les produits d'expression ADAR obtenus à partir d'un échantillon sanguin témoin provenant dudit mammifère.

7. Procédé d'identification in vitro d'un agent qui module l'édition de l'ARNm 5HTR2C chez un mammifère, comprenant les étapes suivantes :
a) de mise en contact d'un échantillon biologique contenant une lignée de cellules sanguines de mammifère en présence d'un modulateur candidat de ladite édition d'ARNm 5HTR2C ; et
b) de détermination des effets dudit modulateur sur les produits d'expression des ADAR ADAR1, isoformes 150 et/ou 110, et les produits d'expression génique ADAR2 exprimés dans ledit échantillon de cellules sanguines, en comparant la quantité des ADAR exprimées obtenues à partir de l'échantillon biologique à l'étape a) avec la quantité des ADAR exprimées obtenues à partir d'un échantillon tissulaire témoin de cellules sanguines dudit mammifère.

8. Procédé destiné à la détermination in vitro chez un patient de l'efficacité d'un médicament utilisé pour la prévention ou pour le traitement d'une pathologie liée à une altération du mécanisme d'édition d'ARNm 5HTR2C, comprenant les étapes suivantes :
a) de détermination des produits d'expression ADAR ADAR1, isoformes 150 et/ou 110, et des produits d'expression génique ADAR2 dans un échantillon sanguin contenant des cellules sanguines obtenues à partir du patient ; et
b) de détermination de l'efficacité dudit médicament en comparant les produits d'expression ADAR ADAR1, isoformes 150 et/ou 110, et les produits d'expression génique ADAR2 obtenus à l'étape a) avec les produits d'expression ADAR obtenus à partir du patient pendant ou/et après le traitement avec ledit médicament,
dans lequel une modulation des produits d'expression ADAR exprimés aboutissant à des produits d'expression ADAR exprimés proches ou égaux à ceux observés chez des patients normaux étant significatif de l'efficacité du traitement.

9. Procédé destiné à la détermination du fait de savoir si un patient répond ou ne répond pas à un traitement d'une pathologie aboutissant ou provoquant par l'altération du mécanisme d'édition d'ARNm du 5HTR2C, comprenant les étapes suivantes :
a) de détermination des produits d'expression ADAR ADAR1, isoformes 150 et/ou 110, et des produits d'expression génique ADAR2 dans un échantillon sanguin contenant des cellules sanguines prélevées chez le patient avant le début du traitement ;
b) de détermination du fait de savoir si le patient répond ou non au traitement en observant la modification des produits d'expression des ADAR après une période de traitement par comparaison avec la quantité des produits d'expression des ADAR obtenue à l'étape a) avant le début du traitement.

10. Procédé selon les revendications 1 à 9, dans lequel le patient ou le mammifère est un humain, une souris ou un rat.

11. Procédé selon les revendications 1 à 10, dans lequel les produits d'expression ADAR sont les isoformes ADAR1 150 et/ou 110, et les produits d'expression du gène ADAR2 du gène humain codant pour la protéine ADAR1, isoformes 150-kD et/ou 110-kD et la protéine ADAR2.

12. Procédé selon les revendications 1 à 11, dans lequel les produits d'expression ADAR sont les ARNm ADAR.

13. Procédé selon la revendication 12, dans lequel, la détermination de l'ARNm ADAR est effectuée par un procédé qui comprend les étapes suivantes :
A) l'extraction des ARN totaux desdites cellules de l'échantillon sanguin, suivie, le cas échéant, d'une purification des ARNm ;
B) la transcription inverse des ARN extraits à l'étape A) ; et
C) l'amplification par PCR des ADNc obtenus à l'étape B) à l'aide d'au moins un couple d'amorces spécifiques de chacun des ARNm ADAR à quantifier et/ou analyser qualitativement.

14. Procédé selon la revendication 13, dans lequel à l'étape C), la paire d'amorces spécifiques pour l'amplification par PCR de l'ARNm de l'ADAR est choisie dans le groupe constitué de :
- pour l'amplification de l'ARNm de l'isoforme ADAR1-150 humain :
Avant : 5 '-GCCTCGCGGGCGCAATGAATCC-S ' (SEQ ID NO. 41),
Inverse : 5 '-CTTGCCCTTCTTTGCCAGGGAG-S ' (SEQ ID NO. 42) ;
- pour l'amplification de l'ARNm de l'isoforme ADAR1-110 humain :
Avant : 5 '-CGAGCCATCATGGAGATGCCCTCC-3 ' (SEQ ID NO. 43),
Inverse : 5 '-CATAGCTGCATCCTGCTTGGCCAC-S ' (SEQ ID NO. 44) ;
- pour l'amplification de l'ARNm ADAR2 humain :
Avant : 5 '-GCTGCGCAGTCTGCCCTGGCCGC-S ' (SEQ ID NO. 45),
Inverse : 5 '-GTCATGACGACTCCAGCCAGCAC-S ' (SEQ ID NO. 46) ;
- pour l'amplification de l'ARNm de l'isoforme ADAR1-150 de souris :
Avant : 5 '-GTCTCAAGGGTTCAGGGGACCC-S ' (SEQ ID NO. 47),
Inverse : 5 '-CTCCTCTAGGGAATTCCTGGATAC-S ' (SEQ ID NO. 48) ;
- pour l'amplification de l'ARNm de l'isoforme ADAR1-110 de souris :
Avant : 5 '-TCACGAGTGGGCAGCGTCCGAGG-S ' (SEQ ID NO. 49),
Inverse : 5 '-CTCCTCTAGGGAATTCCTGGATAC-3 ' (SEQ ID N. 48) ; et
- pour l'amplification de l'ARNm ADAR2 de souris :
Avant : 5 '-GCTGCACAGTCTGCCTTGGCTAC-S ' (SEQ ID NO. 50),
Inverse : 5 '-GCATAAAGAAACCTGAGCAGGGAC-S ' (SEQ ID NO. 51).

15. Procédé selon les revendications 1 à 14, dans lequel les produits d'expression ADAR sont les protéines ADAR.

16. Procédé selon la revendication 15, dans lequel la détermination des protéines ADAR est effectuée par un procédé qui comprend les étapes suivantes :
A) l'extraction des protéines entières contenues dans lesdits globules sanguins suivie, le cas échéant, par une étape de purification des protéines ; et
B) la détermination de la présence et/ou de la concentration de chaque protéine ADAR contenue dans lesdites cellules de prélèvement sanguin par la mise en oeuvre d'anticorps capables de reconnaître spécifiquement lesdites protéines ADAR.
